(19) Europäisches Patentamt
European Patent Office
Office européen des brevets

(11) Publication number : 0 384 522 B1

(12) # EUROPEAN PATENT SPECIFICATION

(45) Date of publication of patent specification : **13.01.93 Bulletin 93/02**

(21) Application number : **90200348.2**

(22) Date of filing : **16.02.90**

(51) Int. Cl.[5] : **C07D 487/06,** C07D 487/04, A61K 31/55, // (C07D487/06, 243:00, 235:00), (C07D487/04, 243:00, 235:00)

(54) **Antiviral tetrahydroimidazo[1,4]benzodiazepin-2-thiones.**

(30) Priority : **23.02.89 GB 8904108**
**08.09.89 GB 8920354**
**13.09.89 US 406626**

(43) Date of publication of application : **29.08.90 Bulletin 90/35**

(45) Publication of the grant of the patent : **13.01.93 Bulletin 93/02**

(84) Designated Contracting States : **AT BE CH DE DK ES FR GB GR IT LI LU NL SE**

(56) References cited :
**EP-A- 0 107 569**
**EP-A- 0 336 466**
**EUROPEAN JOURNAL OF MEDICINAL CHEMISTRY-CHIMICA THERAPEUTICA, vol. 13, 1978, pages 53-54; P. GENESTE et al.: "Recherches ensérie de l'imidazo-(4,5,1-jk)-benzodiazépine-1,4 et de l'imidazo-(1,5,4-ef)-benzodiazépine-1,5"**

(73) Proprietor : **JANSSEN PHARMACEUTICA N.V.**
**Turnhoutseweg 30**
**B-2340 Beerse (BE)**

(72) Inventor : **Kukla, Michael Joseph**
**1551 Oak Hollow Drive**
**Maple Glen, Pennsylvania (US)**
Inventor : **Breslin, Henry Joseph**
**11974 Muhlenburg Drive**
**Lansdale, Pennsylvania (US)**
Inventor : **Raeymaekers, Alfons Herman Margaretha**
**Aanbeeldstraat 1**
**B-2340 Beerse (BE)**
Inventor : **van Gelder, Josephus Ludovicus Hubertus**
**Bareelstraat 30**
**B-2450 Kasterlee (BE)**
Inventor : **Janssen, Paul Adriaan**
**Antwerpsesteenweg 20**
**B-2350 Vosselaar (BE)**

EP 0 384 522 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Background of the invention

In the Eur. J. Med. Chem. 1978, 13, 53-59, there are described three tetrahydroimidazo[4,5,1-jk][1,4]benzodiazepines. The compounds of the present invention differ therefrom by the fact that the imidazo-moiety is substituted with a thioxo group and that said compounds show antiviral activity.

Description of the invention

The present invention is concerned with tetrahydroimidazo[1,4]benzodiazepin-2-thiones having the formula

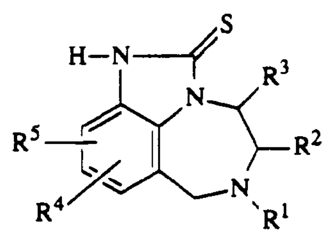

(I),

the pharmaceutically acceptable acid addition salts and the stereochemically isomeric forms thereof, wherein

$R^1$ is $C_{1-6}$alkyl, $C_{3-6}$alkenyl, $C_{3-6}$alkynyl, $C_{3-6}$cycloalkyl, or $C_{1-6}$alkyl substituted with aryl or with $C_{3-6}$cycloalkyl;

$R^2$ is hydrogen or $C_{1-6}$alkyl;

$R^3$ is hydrogen or $C_{1-6}$alkyl;

$R^4$ and $R^5$ each independently are hydrogen, $C_{1-6}$alkyl, halo, cyano, nitro, trifluoromethyl, hydroxy, $C_{1-6}$alkyloxy, amino or mono-or di($C_{1-6}$alkylamino); and

aryl is phenyl optionally substituted with from 1 to 3 substituents independently selected from $C_{1-6}$alkyl, halo, hydroxy, $C_{1-6}$alkyloxy, amino, nitro and trifluoromethyl.

The compounds of formula (I) may also exist in their tautomeric form. Said tautomeric form, although not explicitly indicated in the above formula, is intended to be included within the scope of the present invention.

In the foregoing definitions the term halo is generic to fluoro, chloro, bromo and iodo; $C_{1-6}$alkyl defines straight and branch chained saturated hydrocarbon radicals having from 1 to 6 carbon atoms such as, for example, methyl, ethyl, propyl, 1-methylethyl, butyl, 1-methylpropyl, 2-methylpropyl, 1,1-dimethylethyl, pentyl, hexyl and the like; $C_{3-6}$alkenyl defines straight and branched hydrocarbon radicals containing one double bond and having from 3 to 6 carbon atoms such as, for example, 2-propenyl, 2-butenyl, 3-butenyl, 2-methyl-2-propenyl, pentenyl, hexenyl and the like; $C_{3-6}$alkynyl defines straight and branch chained hydrocarbon radicals containing a triple bond and having from 3 to 6 carbon atoms such as, for example, 2-propynyl, 2-butynyl, 3-butynyl, pentynyl, hexynyl and the like; $C_{3-6}$cycloalkyl defines cyclopropyl, cyclobutyl, cyclopentyl and cyclohexyl.

Depending on the nature of the various substituents, the compounds of formula (I) may have several asymmetric carbon atoms. Unless otherwise mentioned or indicated, the chemical designation of compounds denotes the mixture of all possible stereochemically isomeric forms, said mixtures containing all diastereomers and enantiomers of the basic molecular structure. The absolute configuration of each chiral center may be indicated by the stereochemical descriptors R and S, this R and S notation corresponding to the rules described in Pure Appl. Chem. 1976, 45, 11-30. Stereochemically isomeric forms of the compounds of formula (I) are obviously intended to be embraced within the scope of the invention.

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, liquid chromatography and the like; and enantiomers may be separated from each other by the selective crystallization of their diastereomeric salts with optically active acids. Pure stereochemically isomeric forms may also be derived from the corresponding pure stereochemically isomeric forms of the appropriate starting materials, provided that the reaction occurs stereospecifically.

The compounds of formula (I) have basic properties and, consequently, they may be converted to their therapeutically active non-toxic acid addition salt forms by treatment with appropriate acids, such as, for example, inorganic acids, e.g. hydrochloric, hydrobromic and the like acids, sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form. The term pharmaceutically acceptable acid addition salts also comprises the solvates which the compounds of formula (I) may form and said solvates are intended to be included within the scope of the present invention. Examples of such solvates are e.g. the hydrates, alcoholates and the like.

Particular compounds of formula (I) are those compounds wherein $R^1$ is $C_{1-6}$alkyl, $C_{3-6}$alkenyl, $C_{3-6}$alkynyl or $C_{1-6}$alkyl substituted with aryl or with $C_{3-6}$cycloalkyl; and/or $R^4$ and $R^5$ each independently are hydrogen, $C_{1-6}$alkyl, halo, cyano, nitro, trifluoromethyl, hydroxy or $C_{1-6}$alkyloxy.

More particular compounds are those particular compounds wherein $R^1$ is $C_{3-6}$alkyl, $C_{3-6}$alkenyl, or $C_{1-6}$alkyl substituted with $C_{3-6}$cycloalkyl; and/or $R^2$ is $C_{1-6}$alkyl; and/or $R^5$ is hydrogen.

A first particular subgroup comprises those more particular compounds wherein $R^1$ is $C_{3-6}$alkyl, $C_{3-6}$alkenyl or ($C_{3-6}$cycloalkyl)methyl; and/or $R^2$ is methyl; and/or $R^3$ is hydrogen; and/or $R^4$ is hydrogen, methyl, halo, cyano, nitro or trifluoromethyl.

A second particular subgroup comprises those more particular compounds wherein $R^2$ is methyl; and/or $R^3$ is hydrogen; and/or $R^4$ is hydroxy or $C_{1-6}$alkyloxy.

Particularly interesting compounds within the first particular subgroup are those compounds wherein $R^1$ is propyl, 2-propenyl, 2-butenyl, 2-methyl-2-butenyl, 3-methyl-2-butenyl, 2,3-dimethyl-2-butenyl or cyclopropylmethyl; and/or $R^4$ is hydrogen, methyl or chloro.

The most interesting compounds are 4,5,6,7-tetrahydro-5-methyl-6-propyl imidazo-[4.5.1-jk][1,4]benzodiazepine-2(1H)-thione; (+)-(S)-4,5,6,7-tetrahydro-5-methyl-6-(3-methyl-2-butenyl)imidazo[4,5,1-jk] [1,4]benzodiazepine-2(1H)-thione and (+)-(S)-9-chloro-4,5,6,7-tetrahydro-5-methyl-6-(3-methyl-2-butenyl)imidazo[4,5,1-jk] [1,4]benzodiazepine-2(1H)-thione.

The compounds of formula (I) can generally be prepared by condensing a 9-amino-2,3,4,5-tetrahydro-1H-1,4-benzodiazepine of formula (II) with a reagent of formula (III), wherein L is an appropriate leaving group.

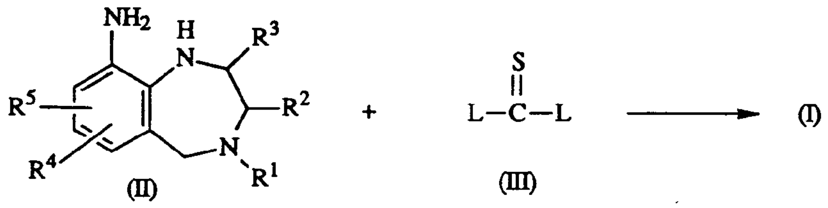

In formula (II) $R^1$, $R^2$, $R^3$, $R^4$ and $R^5$ are as defined in formula (I). Appropriate agents of formula (III) are for example thiourea, carbonothioic dichloride, carbon disulfide, 1,1′-carbonothioylbis[1H-imidazole], xanthogenates, alkali metal, alkaline earth metal or ammonium isothiocyanates, phenylisothiocyanate, benzoyl isothiocyanate, 1,3-dithiolane-2-thione and the like. Said condensation reaction may conveniently be conducted by stirring and optionally heating the reactants in a reaction-inert solvent, preferably having a relatively high boiling point, such as, for example, an aromatic hydrocarbon, e.g. benzene, methylbenzene, dimethylbenzene and the like; a halogenated hydrocarbon, e.g. trichloromethane, tetrachloromethane, chlorobenzene and the like; an ether, e.g. tetrahydrofuran, 1,4-dioxane, 1,1′-oxybisbutane, 1,1′-oxybis(2-methoxyethane), 1,2-bis(2-methoxyethoxy)ethane and the like; a dipolar aprotic solvent, e.g. N,N-dimethylformamide, N,N-dimethylacetamide, dimethyl sulfoxide, 1-methyl-2-pyrrolidinone, pyridine, methylpyridine, dimethylpyridine, tetrahydrothiophene 1,1-dioxide and the like; or a mixture of such solvents. In some instances however, it may be preferable to heat the reactants without a solvent. Further it may be appropriate to add to the reaction mixture a base such as, for example, a tertiary amine, e.g. N,N-diethylethanamine, N-(1-methylethyl)-2-propanamine, 4-methylmorpholine and the like amines. When said reagent of formula (III) is carbon disulfide, the reaction can also be conducted conveniently in an alkanol such as, for example, methanol, ethanol, propanol and the like, in the presence of a base such as sodium or potassium hydroxide and the like, or in carbon disulfide as solvent and in the presence of a suitable base such as, for example, an alkyl magnesium halide, e.g. ethyl magnesium bromide, an alkyl lithium, e.g. butyllithium, an amine, e.g., N,N-diethylethanamine, a carbodiimide, e.g. N,N-dicyclohexylcarbodiimide and the like reagents. Or, alternatively the latter reaction may also be conducted in basic solvent such as, for example, pyridine and the like, in the presence of a phosphite such as for example diphenylphosphite.

The compounds of formula (I) can also be prepared by thionation of a 4,5,6,7-tetrahydroimidazo[4,5,1-jk][1,4]-benzodiazepin-2-one of formula (IV) following art-known carbonyl to thiocarbonyl transformation reactions.

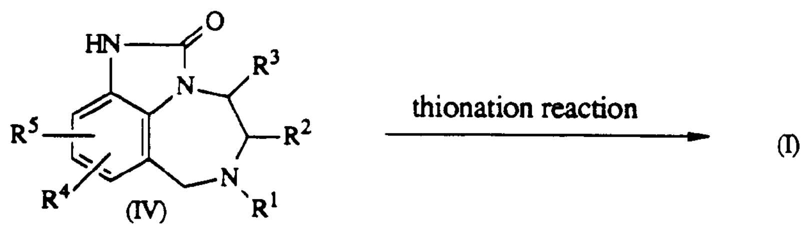


For example, the intermediates of formula (IV) may be reacted with a halogenating reagent such as, for example, phosphoryl chloride, phosphorous trichloride, phosphorous tribromide, thionyl chloride, oxalyl chloride and the like reagents, optionally at an elevated temperature, in particular the reflux temperature of the reaction mixture, and optionally in the presence of a reaction-inert solvent and a base such as, for example, sodium carbonate, sodium hydrogen carbonate, potassium carbonate and the like. The thus obtained 2-halo-4,5,6,7-tetrahydroimidazo[4,5,1-jk][1,4]benzodiazepine can be further converted into the compounds of formula (I) by reaction with thiourea or an alkali metal thiosulfate, e.g. sodium thiosulfate in an appropriate reaction-inert solvent such as for example, water or an alkanol, e.g. methanol, ethanol, 1-propanol, 2-propanol, butanol, 1,2-ethanediol and the like, optionally at an elevated temperature, in particular the reflux temperature of the reaction mixture.

Alternatively, the compounds of formula (I) may also be obtained directly from the intermediates of formula (IV) by thionation with 2,4-bis(4-methoxyphenyl)-1,3-dithia-2,4-diphosphetane-2,4-disulfide (Lawesson's reagent) in an appropriate reaction-inert solvent such as for example, an aromatic hydrocarbon, e.g. benzene, methylbenzene, dimethylbenzene, a dipolar aprotic solvent, e.g. hexamethylphosphoric triamide (HMPA) and the like solvents; or by thionation with phosphorus pentasulfide.

The compounds of formula (I) may also be obtained by N-alkylating an intermediate of formula (V) with a reagent of formula $R^1$-W (VI-a) wherein W represents an appropriate reactive leaving group such as, for example, halo, e.g. chloro, bromo or iodo; or a sulfonyloxy group, e.g. benzenesulfonyloxy, 4-methylbenzenesulfonyloxy, methanesulfonyloxy and the like.

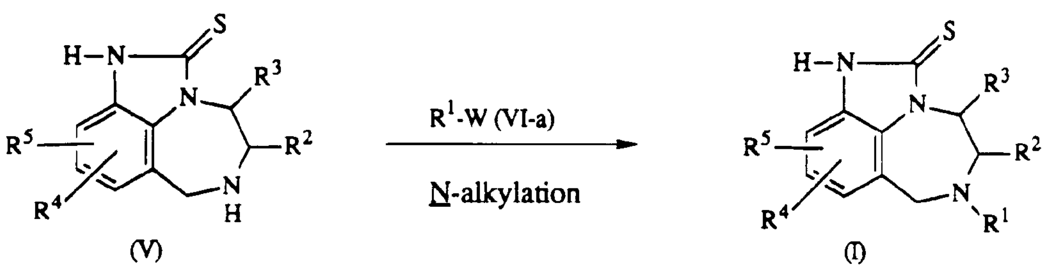


Said N-alkylation reaction may conveniently be conducted in a reaction-inert solvent such as, for example, an aromatic hydrocarbon, e.g., benzene, methylbenzene, dimethylbenzene and the like; a lower alkanol, e.g., methanol, ethanol, 1-butanol and the like; a ketone, e.g., 2-propanone, 4-methyl-2-pentanone and the like; an ether, e.g., 1,4-dioxane, 1,1'-oxybisethane, tetrahydrofuran and the like; a dipolar aprotic solvent, e.g. N,N-dimethylformamide, N,N-dimethylacetamide, nitrobenzene, dimethyl sulfoxide, 1-methyl-2-pyrrolidinone, and the like, or a mixture of such solvents. The addition of an appropriate base such as, for example, an alkali metal carbonate or hydrogen carbonate, e.g. sodium carbonate, sodium hydrogen carbonate; sodium hydride or an organic base such as, for example, N,N-diethylethanamine or N-(1-methylethyl)-2-propanamine and the like may be utilized to pick up the acid which is liberated during the course of the reaction. In some circumstances the addition of an iodide salt, preferably an alkali metal iodide, e.g. potassium iodide, is appropriate. Somewhat elevated temperatures and stirring may enhance the rate of the reaction.

The compounds of formula (I) wherein $R^1$ is other than $C_{3-6}$alkenyl or $C_{3-6}$alkynyl and the carbon atom of

said $R^1$ radical adjacent to the nitrogen atom bearing said $R^1$ contains at least one hydrogen atom, said radicals being represented by $R^{1-a}$, and said compounds by formula (I-a), may also be prepared by the reductive N-alkylation of an intermediate of formula (V) with a ketone or aldehyde of formula $R^{1-b}$=O (VI-b). In formula (VI-b), $R^{1-b}$ represents a geminal bivalent radical derived from $R^{1-a}$-H wherein two geminal hydrogen atoms are replaced by =O.

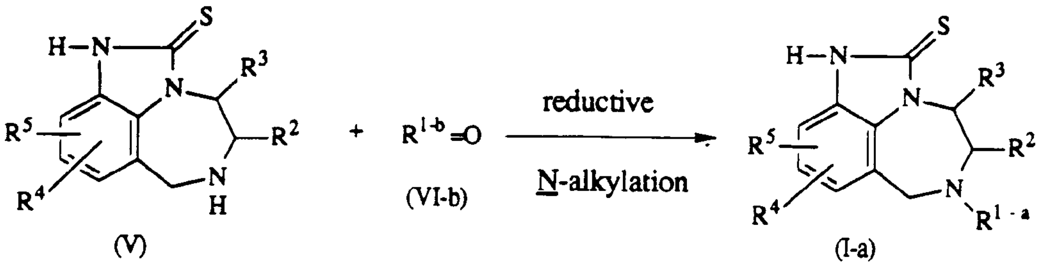

(V) (VI-b) (I-a)

Said reductive N-alkylation reaction may conveniently be carried out by catalytically hydrogenating the reactants in a suitable reaction-inert organic solvent according to art-known catalytic hydrogenation procedures. The reaction mixture may be stirred and/or heated in order to enhance the reaction rate. Suitable solvents are, for example, water; $C_{1-6}$alkanols, e.g. methanol, ethanol, 2-propanol and the like; ethers, e.g. 1,4-dioxane and the like; halogenated hydrocarbons, e.g. trichloromethane and the like; dipolar aprotic solvents, e.g. N,N-dimethylformamide, dimethyl sulfoxide and the like; esters, e.g. ethyl acetate and the like; or a mixture of such solvents. The term "art-known catalytic hydrogenation procedures" means that the reaction is carried out under a hydrogen atmosphere and in the presence of an appropriate catalyst such as, for example, palladium-on-charcoal, platinum-on-charcoal and the like. In order to prevent the undesired further hydrogenation of certain functional groups in the reactants and the reaction products it may be advantageous to add an appropriate catalyst-poison to the reaction mixture, e.g., thiophene and the like. Alternatively, said reductive N-alkylation may also be performed following art-known reduction procedures by treating a stirred and, if desired, heated mixture of the reactants with a reducing agent such as, for example, sodium borohydride, sodium cyanoborohydride, formic acid or a salt thereof, in particular the ammonium salt thereof.

The compounds of formula (I) may also be obtained by direct thiation of a tetrahydroimidazo[4,5,1-jk][1,4]benzodiazepine of formula (VII) with elemental sulfur at an elevated temperature.

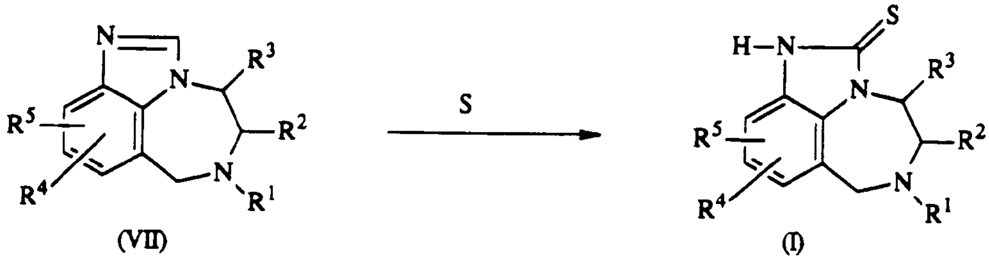

(VII) (I)

Said reaction may conveniently be conducted without a solvent at a temperature above 200°C, more particularly a temperature in the range of 230 to 250°C.

The compounds of formula (I) may also be prepared by the combined reduction-thiocarbonylation of a 9-nitrobenzodiazepine of formula (VIII) in the presence of an alkali metal sulfide or hydrogen sulfide, and carbon disulfide.

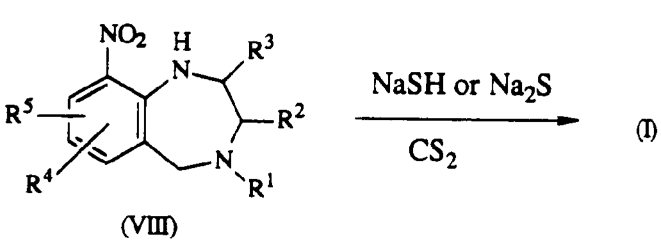

(VIII) (I)

Said reduction-thiocarbonylation reaction may conveniently be conducted by stirring the reactants in a reaction-inert solvent, optionally at an elevated temperature.

The compounds of formula (I) may also be prepared by cyclizing a benzimidazol-2-thione of formula (IX) in a suitable reaction-inert solvent, optionally in the presence of a base and optionally at an elevated temperature.

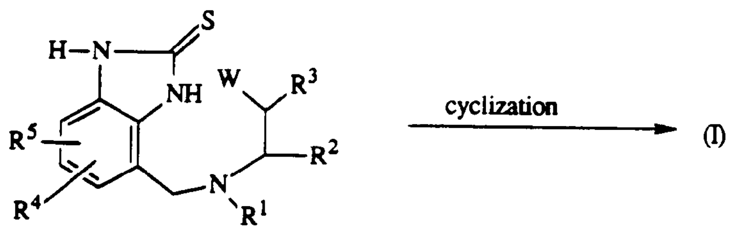


(IX)

In formula (IX), W represents a reactive leaving group as defined hereinbefore. Said cyclization reaction may conveniently be conducted by stirring, and, if desired, heating the starting material. Suitable solvents are, for example, aromatic hydrocarbons, e.g. benzene, methylbenzene, dimethylbenzene and the like, halogenated hydrocarbons, e.g. trichloromethane, tetrachloromethane, chlorobenzene and the like, ethers, e.g. tetrahydrofuran, 1,4-dioxane and the like, dipolar aprotic solvents e.g. N,N-dimethylformamide, N,N-dimethylacetamide, acetonitrile, dimethylsulfoxide, pyridine and the like. Bases which may conveniently be employed in said cyclization reaction are, for example, alkali metal or earth alkaline metal carbonates, hydrogen carbonates, hydroxides, oxides, amides, hydrides and the like. In some instances the addition to the reaction mixture of a iodide salt, preferably an alkali metal iodide, e.g. potassium iodide, may be advantageous.

In all of the foregoing and in the following preparations, the reaction products may be isolated from the reaction mixture and, if necessary, further purified according to methodologies generally known in the art.

A number of intermediates and starting materials in the foregoing preparations are known compounds which may be prepared according to art-known methodologies of preparing said or similar compounds and some intermediates are new. A number of such preparation methods will be described hereinafter in more detail.

The intermediates of formula (II) can generally be prepared from a 9-aminobenzodiazepine of formula (II-a) following N-alkylation reaction procedures such as described hereinabove for the preparation of the compounds of formula (I) from an intermediate of formula (V) with an alkylating reagent (VI-a) or with an aldehyde or ketone of formula (VI-b).

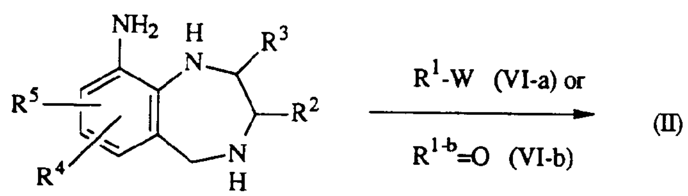


(II-a)

In order to simplify the following reaction schemes, the N-alkylated intermediates wherein $R^1$ is as defined under formula (I) and the $N^4$-unsubstituted intermediates (wherein $R^1$ is replaced by hydrogen) will be represented hereinafter by formulae wherein $N^4$ is substituted with $R^{1H}$, said $R^{1H}$ defining $R^1$ and hydrogen. In intermediates (X), (XI), (XIII), (XIV), (XVI) and (XVII) of scheme 1 hereinbelow, $R^{1H}$ also defines $C_{1-5}$alkylcarbonyl, arylcarbonyl, ($C_{3-6}$cycloalkyl)carbonyl or [(aryl)- or ($C_{3-6}$cycloalkyl)]$C_{1-5}$alkylcarbonyl. Said intermediates can conveniently be prepared following art-known N-acylation procedures from corresponding intermediates wherein $R^{1H}$ is hydrogen and can be reduced to the corresponding N-alkylated intermediates with complex metal hydrides or hydrides as described under reaction step A of scheme 1. In all of the following reaction schemes, the intermediates wherein $R^{1H}$ is hydrogen can also be converted into intermediates wherein $R^{1H}$ is $R^1$ following the above described N-alkylation procedures.

The intermediates of formula (II-H), said intermediates representing the intermediates of formula (II) and (II-a) can generally be prepared following the reaction steps shown in the reaction scheme 1 below.

Scheme 1

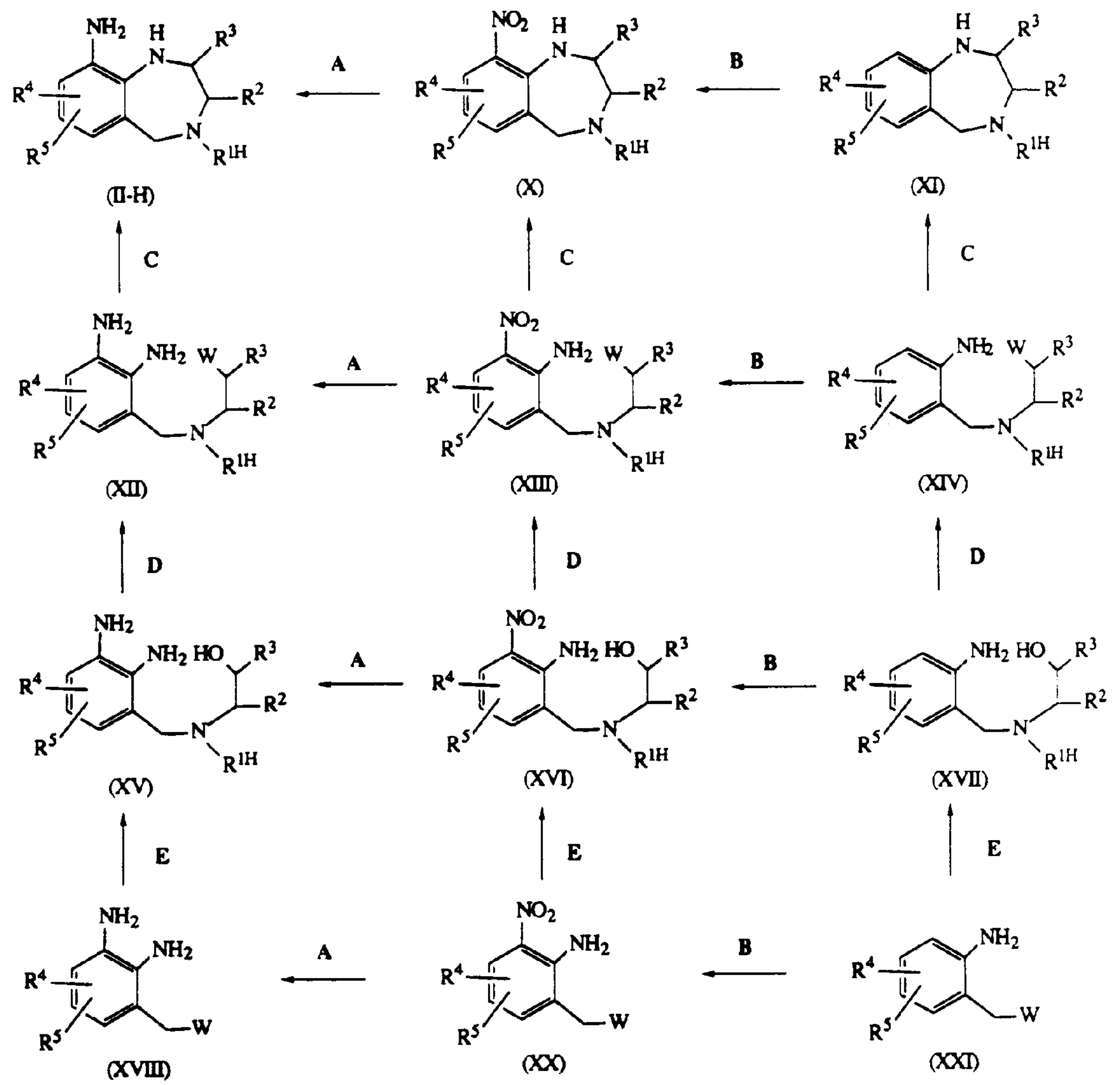


(II-H) (X) (XI) (XII) (XIII) (XIV) (XV) (XVI) (XVII) (XVIII) (XX) (XXI)

**A** : nitro-to-amine reduction

**B** : nitration

**C** : cyclization

**D** : -OH-to-W activation

**E** : N-alkylation : $R^{1H}NH-CH(R^2)-CH(R^3)OH$ (XIX)

The aniline derivatives in the above reaction scheme may conveniently be prepared by reduction of the corresponding nitrobenzene derivatives following art-known nitro-to-amine reduction procedures (reaction step A). Said reduction may conveniently be conducted by treatment of said nitrobenzenes with a reducing agent such as, for example, a complex metal hydride, e.g. lithium aluminum hydride; a hydride, e.g. diborane, aluminum hydride and the like, in a reaction-inert solvent such as, for example, 1,1'-oxybisethane, tetrahydrofuran, 1,4-dioxane, 1,2-dimethoxyethane and the like, optionally in the presence of a cosolvent such as an aromatic hydrocarbon, e.g. benzene, methylbenzene and the like, and, if desired, at an elevated temperature. Alternatively, said reduction may also be accomplished by treatment of said nitrobenzene derivatives with sodium dithionite, sodium sulfide, sodium hydrogen sulfide, titanium(III) chloride and the like reducing agents in a suitable solvent, in particular water.

Said nitro-to-amine reduction may also be conducted following art-known catalytic hydrogenation procedures. For example, said reduction may be carried out by stirring the reactants under a hydrogen atmosphere and in the presence of an appropriate catalyst such as, for example, palladium-on-charcoal, platinum-on-charcoal, Raney-nickel and the like catalysts. Suitable solvents are, for example, water, alkanols, e.g. methanol, ethanol and the like, esters, e.g. ethyl acetate and the like. In order to enhance the rate of said reduction reaction it may be advantageous to elevate the temperature and/or the pressure of the reaction mixture. Undesired further hydrogenation of certain functional groups in the reactants and the reaction products may be prevented by the addition of a catalyst poison such as, for example, thiophene and the like, to the reaction mixture.

The nitrobenzene derivatives in the above reaction scheme 1 can be prepared from benzenamine derivatives following art-known nitration procedures (reaction step B). For example, the starting materials may be nitrated by treatment with concentrated or fuming nitric acid in the presence of concentrated sulfuric acid and optionally in the presence of a cosolvent such as, for example, a halogenated hydrocarbon, e.g. dichloromethane, trichloromethane, tetrachloromethane and the like solvents. Alternatively, said nitration may in some instances also be accomplished by adding the nitrate salt of the starting material to concentrated sulfuric acid.

The benzodiazepine derivatives (II-H), (X) and (XI) may be obtained from the corresponding aniline derivatives (XII), (XIII) and (XIV) following the cyclization procedures such as described hereinabove for the preparation of the compounds of formula (I) from intermediates of formula (IX) (Reaction step C). Said aniline derivatives in turn, wherein W is a reactive leaving group as defined hereinbefore, may be prepared from the corresponding alkanols by treatment with a halogenating reagent such as, for example, thionyl chloride, phosphoryl chloride, phosphorous trichloride and the like; or by treatment with a sulfonylating reagent, e.g. methanesulfonyl chloride, 4-methylbenzenesulfonyl chloride and the like (Reaction step D). Said alkanols may be prepared by N-alkylating appropriately substituted benzene derivatives of formulae (XVIII), (XX) or (XXI) with an aminoethanol derivative of formula $R^{1H}NH-CH(R^2)-CH(R^3)OH$ (XIX) following art-known N-alkylation procedures such as described hereinabove (Reaction step E).

The intermediates of formula (II-H) may also be obtained following the reaction steps shown in reaction scheme 2 below. Reaction steps designated A through D are intended to refer back to the analogous reaction steps described in the previous reaction scheme.

For example, the intermediates of formula (II-H) can also be prepared from an 9-amino- or a 9-nitrobenzodiazepin-5-one of formula (XXII) or (XXIII) by reduction with a complex metal hydride e.g. lithium aluminum hydride and the like in a suitable reaction-inert solvent such as, for example, 1,2-dimethoxyethane, 1,1′-oxybis(2-methoxyethane), 2,5,8,11-tetraoxadodecane, methoxybenzene and the like solvents (Reaction steps F and G). In order to enhance the rate of said reduction reaction it may be advantageous to employ an excess of the reducing reagent and to conduct said reaction at an enhanced temperature of the reaction mixture, in particular the reflux temperature of the reaction mixture.

The intermediates of formula (XXIII) can alternatively be obtained from an appropriately substituted nitrobenzene (XXXIV) by a condensation reaction (reaction step H) with a diamino reagent $R^{1H}NH-CH(R^2)-CH(R^3)-NH_2$ of formula (XXXV) in a suitable reaction-inert solvent such as, for example, an alkanol, e.g. methanol, ethanol, 2-propanol, 1-butanol and the like; an aromatic hydrocarbon, e.g. benzene, methylbenzene, dimethylbenzene and the like; a halogenated hydrocarbon, e.g. trichloromethane, tetrachloromethane and the like; an ether, e.g. tetrahydofuran, 1,4-dioxane, 1,1′-oxybisbutane, 1,1′-oxybis(2-methoxyethane) and the like; a ketone, e.g. 2-propanone, 4-methyl-2-pentanone and the like; a dipolar aprotic solvent, e.g. N,N-dimethylformamide, N,N-dimethylacetamide, dimethyl sulfoxide and the like; or a mixture of such solvents. It may be appropriate to add a base such as an alkali metal or earth alkaline metal carbonate, e.g. sodium carbonate, sodium hydrogen carbonate and the like, to the reaction mixture. Said condensation reaction can conveniently be conducted at an elevated temperature, in particular at the reflux temperature of the reaction mixture.

Scheme 2

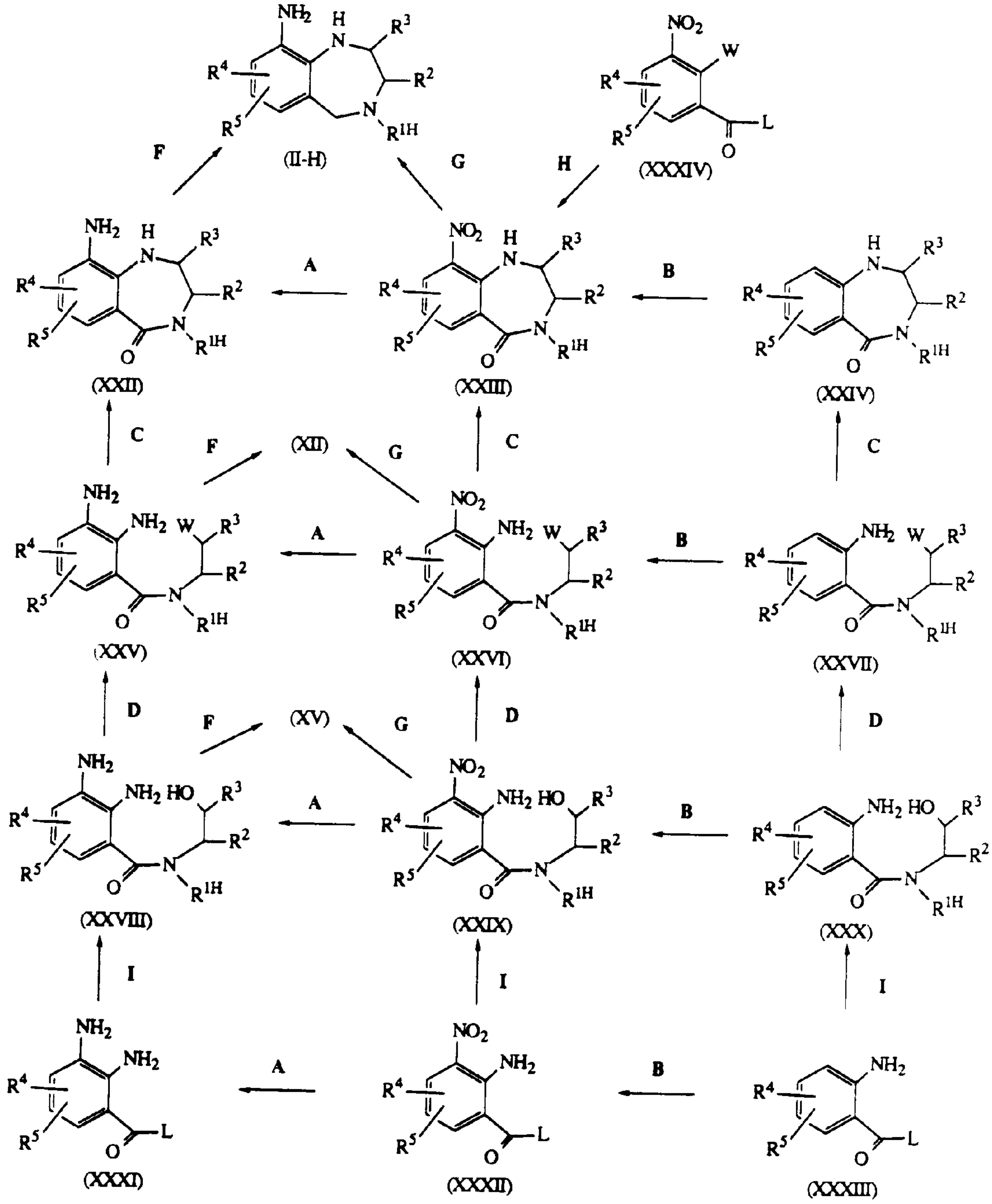


**F** : amide-to-amine reduction

**G** : (nitro-to-amino) and (amide-to-amine) reduction

**H** : cyclization; $R^{1H}$-NH-CH($R^2$)-CH($R^3$)-$NH_2$ (XXXV)

**I** : N-acylation reaction

The amide derivatives (XXVIII), (XXIX) and (XXX) in the above reaction scheme can conveniently be prepared

by N-acylating an ethanolamine of formula $R^{1H}NH-CH(R^2)-CH(R^3)-OH$ (XIX) with an appropriately substituted 2-aminobenzoic acid derivative of formula (XXXI), (XXXII) or (XXXIII) wherein L represents hydroxy or a leaving group such as, for example, halo, e.g. chloro or bromo, alkylcarbonyloxy, e.g. acetyl, alkyloxy, e.g. methoxy, ethoxy and the like, or imidazolyl and the like leaving groups. Said N-acylation reaction (reaction step I) may be carried out by stirring the reactants in a reaction-inert solvent, optionally at an elevated temperature. In those instances where L represents hydroxy, said N-acylation reaction may also be carried out by treating the reactants with reagents capable of forming amides such as, for example, N,N-dicyclohexylcarbodiimide (DCC) optionally in the presence of a catalyst such as hydroxybenzotriazole (HOBT) or 4-dimethylaminopyridine (DMAP); 2-chloro-1-methylpyridinium iodide, 1,1'-carbonylbis[1H-imidazole], 1,1'-sulfonylbis[1H-imidazole] and the like reagents. Suitable solvents are halogenated hydrocarbons, e.g. dichloromethane, trichloromethane and the like, ethers, e.g. tetrahydrofuran, 1,4-dioxane and the like, dipolar aprotic solvents, e.g. N,N-dimethylformamide, N,N-dimethylacetamide, pyridine and the like; or mixtures of such solvents.

The intermediates of formula (II-H) wherein $R^3$ is hydrogen, said intermediates being represented by formula

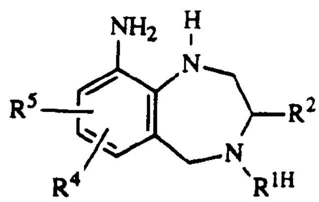

(II-H-α),

can also be prepared from a benzodiazepinedione of formula

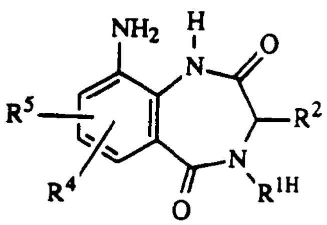

(XXXVI),

following the reduction procedures as described hereinabove for converting intermediates (XXII) or (XXIII) into intermediate (II-H). The preparation of the intermediates of formula (XXXVI) may generally be conducted following the reaction pathways described in scheme 3 hereinbelow.

In all of the following reaction schemes, those compounds wherein $R^3$ is hydrogen, are designated by appending the suffix -α to their numerical reference.

Scheme 3

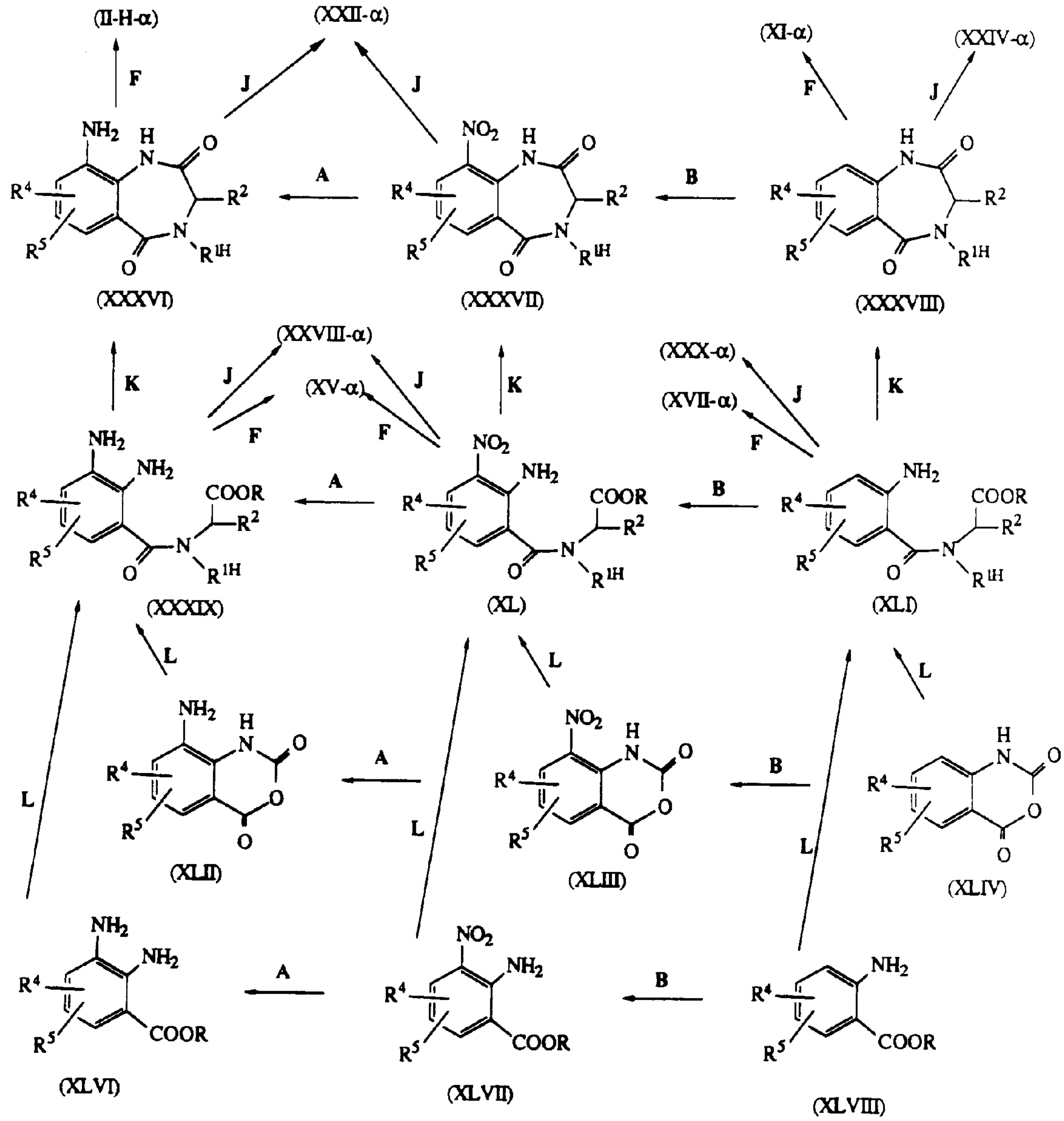


J : (nitro-to-amino) and aliphatic amide-to-amine reduction

K : cyclization to benzodiazepinedione

L : N-acylation of $R^{1H}NH-CH(R^2)-COOR$ (XLV)

In a number of the intermediates shown in scheme 3, for example in (XXXVI), (XXXVII), (XXXVIII), (XXXIX), (XL) and (XLI) it is further possible to selectively reduce functional groups such as the nitro group, the ester group and/or the aliphatic amide group, in the presence of the aromatic amide group (reaction step J). Said selective reduction may be carried out by treatment of the appropriate starting material with a complex metal hydride such as, for example, lithium aluminum hydride in a reaction-inert solvent such as, for example, tetrahydrofuran, 1,4-dioxane and the like. Alternatively, said selective reduction may also be performed by treatment of the appropriate starting material with sodium bis(2-methoxyethoxy) aluminum hydride, or with sodium borohydride in the presence of a suitable metal salt such as, for example, calcium chloride, cerium(III) chloride, aluminum chloride, zirconium(IV) chloride and the like metal salts, in a reaction-inert solvent, in particular an ether.

The benzodiazepinediones in scheme 3 can be obtained by cyclizing (reaction step K) the corresponding acyclic intermediates of formula (XXXIX), (XL) and (XLI), wherein R represents a group such as $C_{1-6}$alkyl or aryl,

a) by heating without a solvent under an inert atmosphere, optionally under reduced pressure;

b) by treating with a bifunctional catalyst such as, for example, acetic acid, 2-hydroxypyridine, pyrazole, 1,2,4-triazole and the like, in a reaction-inert solvent such as, for example, an aromatic hydrocarbon, e.g. methylbenzene, dimethylbenzene and the like, optionally at an elevated temperature; or

c) by hydrolyzing the ester and subsequently treating the corresponding carboxylic acid (R = H) with an appropriate acid, such as, for example, a hydrohalic acid, e.g. hydrochloric acid; sulfuric acid, phosphoric acid and the like acids; or with a halogenating reagent such as, for example, thionyl chloride and the like.

Said intermediates in turn, can be prepared from an appropriately protected amino acid of formula $R^{1H}$-NH-CH($R^2$)-COOR (XLV) wherein R is $C_{1-6}$alkyl or aryl, by a N-acylation reaction (reaction step L) with an appropriately substituted isatoic anhydride derivative or an appropriate 2-aminobenzoic acid derivative, by stirring the reactants at reflux temperature in an reaction-inert solvent such as, for example, trichloromethane, pyridine and the like solvents.

The intermediates of formula (II-H-α) may alternatively be prepared from benzodiazepin-2-one derivatives following the procedures described in scheme 4.

Scheme 4

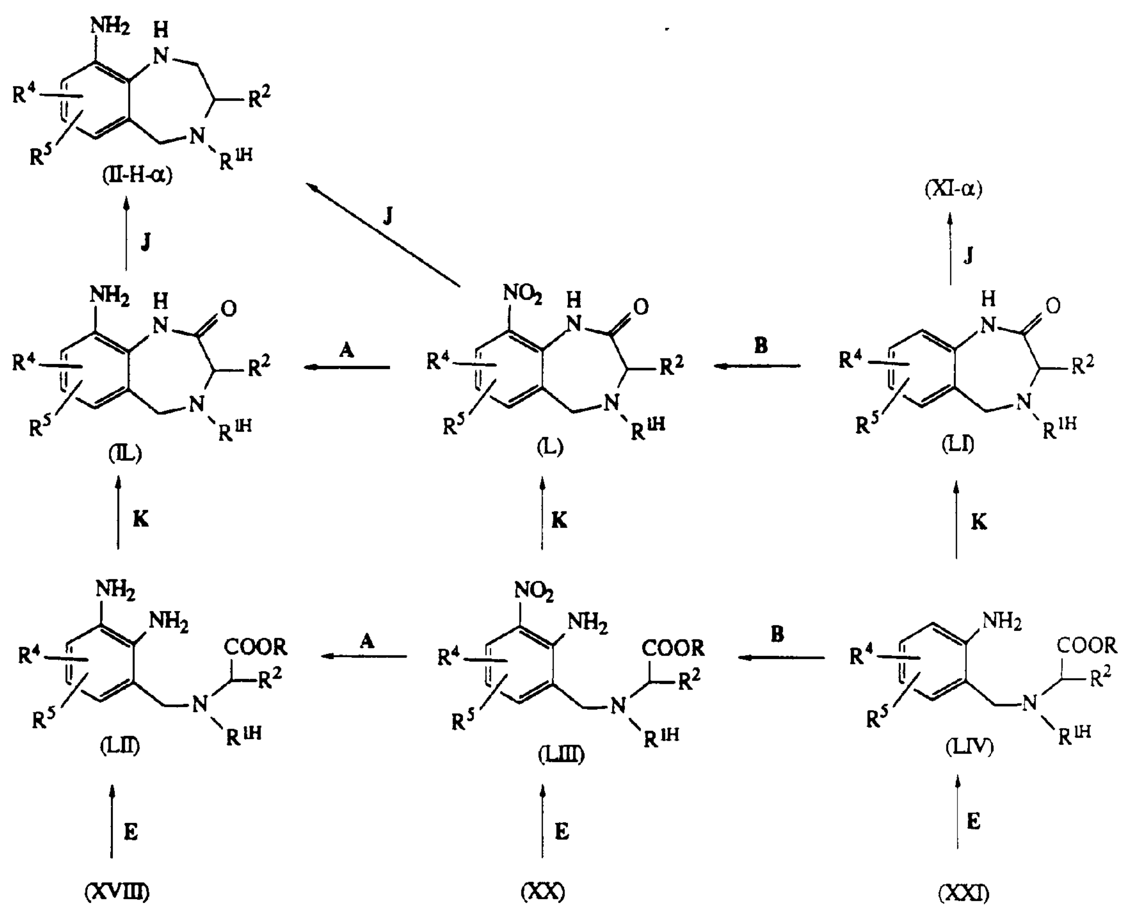


The intermediates of formula (II-H) wherein $R^3$ is $C_{1-6}$alkyl, said radical being represented by $R^{3-a}$ and said

intermediates by formula

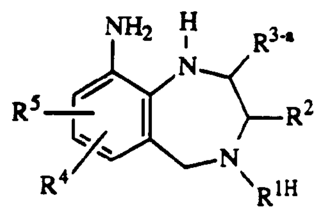

(II-b)

can be prepared by the reduction of an amine (XXII-b) or an imine (LV), following the reduction procedures as described hereinabove for the preparation of (II-H) from (XXII) or (XXIII).

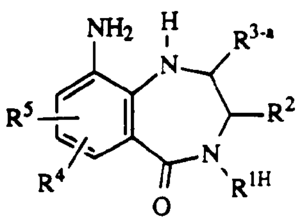

(XXII-b)

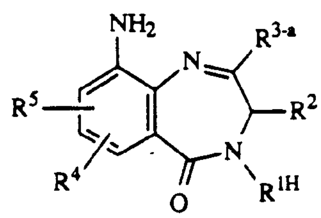

(LV)

The imine (LV) can be prepared by reducing a nitro derivative (LVI) in the presence of hydrogen and a suitable metal catalyst such as, for example, palladium-on-charcoal, platinum oxide and the like catalysts. The ketone of formula (LVI) in turn can be prepared from a 2-amino-3-nitrobenzoic acid or a functional derivative thereof (XXXII) and an α-aminoketone (LVII) following art-known N-acylation procedures.

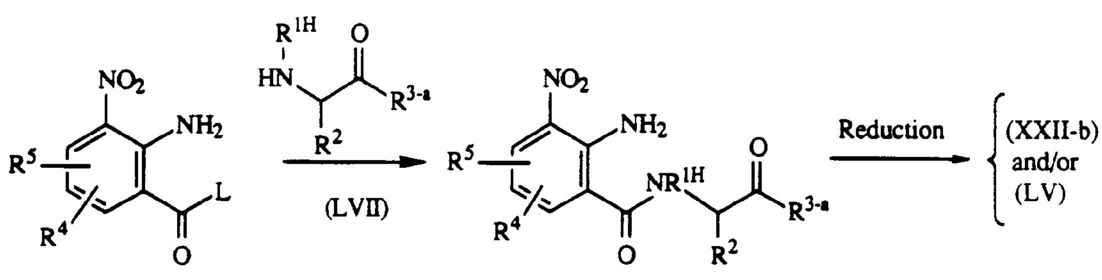


(XXXII) (LVI)

The intermediates of formula (IV) wherein $R^1$, $R^2$ and $R^3$ are as defined under formula (I) and

(a) $R^4$ and $R^5$ each independently are $C_{1-6}$alkyl, halo, cyano, nitro, trifluoromethyl, hydroxy, $C_{1-6}$alkyloxy, amino- or mono- or di($C_{1-6}$alkyl)amino, or

(b) $R^4$ is hydrogen and $R^5$ is cyano, nitro, trifluoromethyl, hydroxy, $C_{1-6}$alkyloxy, amino or mono- or di($C_{1-6}$alkyl)amino;

said radicals $R^4$ and $R^5$ being represented respectively by the radicals $R^{4-a}$ and $R^{5-a}$, and said intermediates by formula (IV-a), are novel.

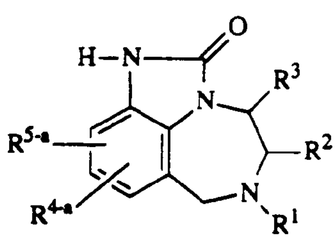

(IV-a)

The intermediates of formula (IV) can be prepared following the procedures described in EP-A-0,336,466, incorporated herein by reference. For example, said intermediates of formula (IV), both known and new ones, can be prepared by N-alkylating an intermediate of formula (IV-b) following art-known N-alkylation procedures such as described hereinabove for the transformation of the intermediates (V) into compounds of formula (I).

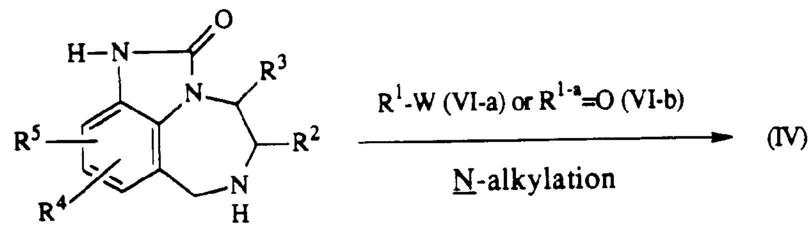


(IV-b)

The intermediates of formula (IV) and (IV-b) can be prepared from an intermediate of formula (II-H) by reaction with a reagent of formula (LVIII) wherein L is an appropriate leaving group.

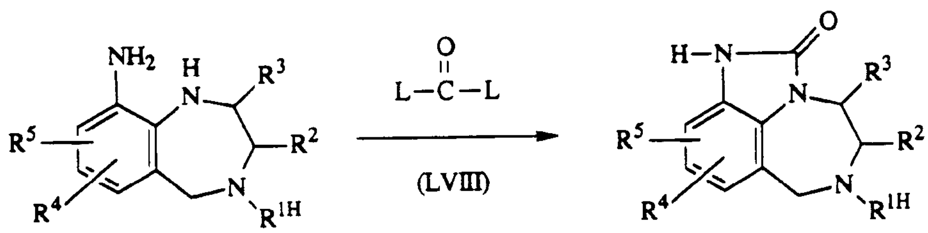


(II-H)

(IV) ($R^{1H} = R^1$)
(IV-b) ($R^{1H}$ = H)

Appropriate agents of formula (LVIII) are, for example, urea, di($C_{1-6}$alkyl)carbonate, carbonoic dichloride, trichloromethyl chloroformate, 1,1′-carbonylbis[1H-imidazole], ethyl chloroformate and the like. Said reaction may conveniently be conducted following the procedures described hereinabove for the conversion of the intermediates of formula (II) into the compounds of formula (I).

The intermediates of formula (V), the acid addition salt forms and the stereochemically isomeric forms thereof are novel and can be obtained from a benzylated compound of formula (I-b) following art-known hydrogenolysis procedures.

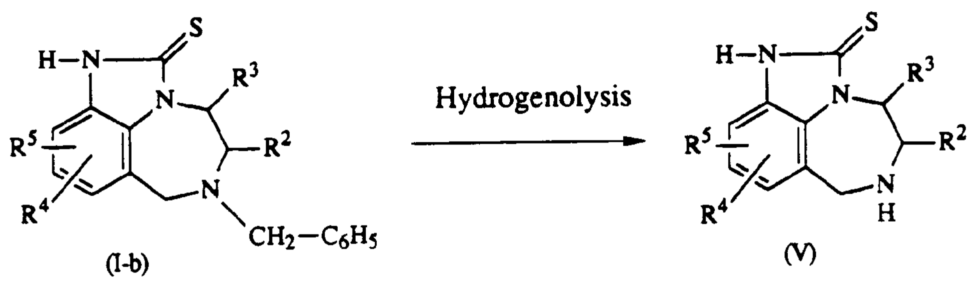


(I-b) (V)

In formulae (V) and (I-b), $R^2$, $R^3$, $R^4$ and $R^5$ have the previously defined meaning. Said debenzylation reaction can be accomplished by stirring a compound of formula (I-b) in an appropriate reaction-inert solvent in the presence of a suitable metal catalyst and under a hydrogen atmosphere. Appropriate solvents are, for example, alkanols, e.g. methanol, ethanol and the like; carboxylic esters, e.g. ethyl acetate; carboxylic acids, e.g. acetic acid, propanoic acid and the like. As examples of suitable metal catalysts there may be mentioned palladium-on-charcoal, platinum-on-charcoal and the like catalysts. In order to prevent the further hydrogenation of the starting material and/or the reaction product it may be appropriate to add a catalyst-poison to the reaction mixture such as, for example thiophene.

The intermediates of formula (V) may also be prepared by thionation of an intermediate of formula (IV-b) following the procedures described hereinabove for the preparation of the compounds of formula (I) from the

intermediates of formula (IV).

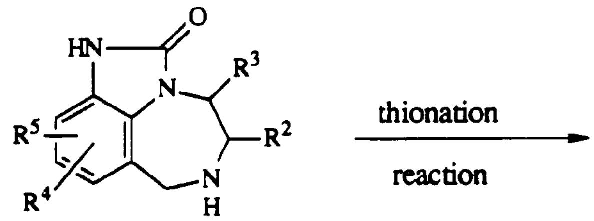


(V)

(IV-b)

Alternatively, said intermediates (V) may also be obtained from an intermediate (II-a) following the condensation reaction with a reagent of formula L-C(=S)-L (III) as described hereinabove for the preparation of the compounds of formula (I) from the intermediates of formula (II).

In all of the foregoing reaction schemes, the chemical designation of the intermediates defines the mixture of all possible stereochemically isomeric forms; mixtures of a number of possible stereochemically isomeric forms such as, for example, diastereomeric mixtures, enantiomeric mixtures, e.g. racemates and enriched enantiomeric mixtures; and the enantiomerically pure isomeric forms of the basic molecular structure.

Stereochemically isomeric forms of the intermediates described in the foregoing reaction schemes and of the compounds of formula (I) may be obtained by the application of art-known procedures. For example, diastereoisomers may be separated by physical separation methods such as destillation, selective crystallization, chromatographic techniques, e.g. counter current distribution, liquid chromatography and the like techniques. Enantiomerically pure intermediates can conviently be obtained from the enantiomerically pure isomeric forms of the appropriate starting materials, provided that the subsequent reactions occur stereospecifically. Particularly interesting enantiomerically pure starting materials for use in the foregoing reaction schemes are aminoacids and/or substituted derivatives thereof, having the formula $R^{1H}NH\text{-}CHR^2\text{-}COOR$ (XLV), and the corresponding aminoalkanols and/or substituted derivatives thereof, having the formula

$R^{1H}NH\text{-}CHR^2\text{-}CH(R^3)OH$ (XIX).

Alternatively, enantiomerically pure intermediates may also be obtained by separating the corresponding racemates for example, by the selective crystallization of their diastereomeric salts with optically active resolving agents, chromatography of diastereomeric derivates, chromatography of the racemate over a chiral stationary phase and the like techniques.

The compounds of formula (I) and the intermediates of formula (IV-a) show antiviral and in particular antiretroviral properties. Until recently, retroviruses were considered to be the pathogenic agents in a number of non-human warm-blooded animal diseases only, unlike viruses which have been known for quite some time to be the cause of a large number of diseases in warm-blooded animals and humans alike. However, since it has been established that a retrovirus, Human Immunodeficiency Virus (HIV), also known as LAV, HTLV-III or ARV, is the etiological agent of Acquired Immune Deficiency Syndrome (AIDS) in humans, retroviral infections and the treatment of subjects suffering therefrom have received the utmost attention. The HIV virus preferentially infects human T-4 cells and destroys them or changes their normal function, particularly the coordination of the immune system. As a result, an infected patient has an everdecreasing number of T-4 cells, which moreover behave abnormally. Hence, the immunological defense system is unable to combat infections and neoplasms and the HIV infected subject usually dies by opportunistic infections such as pneumonia, or by cancers, rather than as a direct result of HIV infections. Other conditions associated with HIV infection include thrombocytopaenia, Kaposi's sarcoma and infection of the central nervous system characterized by progressive demyelination, resulting in dementia and symptoms such as, progressive dysarthia, ataxia and disorientation. HIV infection further has also been associated with peripheral neuropathy, progressive generalized lymphadenopathy (PGL) and AIDS-related complex (ARC). The antiviral, in particular antiretroviral and especially the anti-HIV properties of the compounds of formula (I) and the intermediates of formula (IV-a) suggest said compounds and intermediates to be useful antiviral chemotherapeutical agents for the prophylaxis or treatment of warm-blooded animals suffering from viral infections, more particularly for the treatment of humans infected by HIV virus, especially HIV-1.

Due to their antiviral and in particular their antiretroviral properties, the compounds of formula (I) and the intermediates of formula (IV-a), their pharmaceutically acceptable salts and the stereochemically isomeric forms thereof, are useful in the treatment of warm-blooded animals infected with viruses, in particular retroviruses or for the prophylaxis of said warm-blooded animals. Examples of human retroviral infections include HIV in particular HIV-1 and HTLV-I (human T-lymphotropic virus type I), causing leukemia and lymphoma. As

an example of non-human animal retroviral infection there may be mentioned FeLV (feline leukemia virus) which causes leukemia and immunodeficiency. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic CNS diseases caused by retroviruses, such as, for example HIV mediated dementia and multiple sclerosis.

In view of their antiviral, in particular antiretroviral activity, the subject compounds may be formulated into various pharmaceutical forms for administration purposes. To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) and (IV-a) due to their increased water solubility over the corresponding base form, are obviously more suitable in the preparation of aqueous compositions. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The present invention is also related with a method of treating viral diseases in warm-blooded animals suffering from said viral diseases by administering an effective antiviral amount of a compound of formula (I) or an intermediate of formula (IV-a), a pharmaceutically acceptable acid addition salt or a stereoisomeric form thereof. Those of skill in the treatment of viral diseases could easily determine the effective antiviral amount from the test results presented herein. In general it is contemplated that an effective amount would be from 0.01 mg/kg to 20 mg/kg body weight, and in particular from 0.1 mg/kg to 5 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The following examples are intended to illustrate and not to limit the scope of the present invention in all its aspects. Unless otherwise stated all parts therein are by weight.

## Experimental part

### A. Preparation of the intermediates

#### Example 1

a) To a stirred solution of 11.8 parts of 1-propanamine in 24.9 parts of 1,1′-oxybisethane was added a solution of 18.1 parts of ethyl 2-bromopropanoate in 24.9 parts of 1,1′-oxybisethane at room temperature during 3 minutes. After stirring for 72 hours at room temperature, the reaction mixture was filtered and the filtrate was rinsed with a small amount of 1,1′-oxybisethane. The combined 1,1′-oxybisethane layers were evaporated under reduced pressure, yielding 15.9 parts (100%) of ethyl N-propylalanine as a residue (interm. 1).

b) A mixture of 9.90 parts of 2-amino-3-nitrobenzoic acid and 32.4 parts of thionyl chloride was stirred for 15 minutes at reflux temperature under argon atmosphere. After 15 minutes, the excess of thionyl chloride was removed under reduced pressure, yielding 10.8 parts (100%) of 2-amino-3-nitrobenzoyl chloride as a residue (interm. 2).

c) To a stirred and cooled (0°C) solution of 8.65 parts of intermediate 1 and 5.52 parts of N,N-diethylethanamine in 53.2 parts of dichloromethane was added over a period of 10 minutes a suspension of 10.83 parts of intermediate 2 in 119.7 parts of dichloromethane under an argon atmosphere. After 5 minutes, the ice-bath was removed and stirring was continued for 1 hour. The reaction mixture was extracted successively with water, $NaHCO_3$ solution (sat.), citric acid 2N and again $NaHCO_3$ solution (sat.). The organic layer was dried, filtered and evaporated, yielding 19.14 parts (100%) of ethyl N-(2-amino-3-nitrobenzoyl)-N-propylalanine (interm. 3).

d) A solution of 17.5 parts of intermediate 3 in 80 parts of ethanol was hydrogenated at 3.5 $10^5$ Pa and at room temperature with 2.0 parts of palladium-on-charcoal catalyst 10%. After the calculated amount of hydrogen was taken up, the catalyst was filtered off over diatomaceous earth and the filtrate was concentrated under reduced pressure. The resulting oil was heated under vacuo (3.3 $10^3$ Pa) in an oil bath at 100°C for 1.5 hours. After cooling, the oil was purified by column chromatography (silica gel; $CH_2Cl_2/CH_3OH$ 20:1), yielding 4.6 parts (34.4%) of 9-amino-3-methyl-4-propyl-3H-1,4-benzodiazepine-2,5(1H,4H)-dione as a residue (interm. 4).

e) To a stirred and cooled (0°C) suspension of 1.28 parts of lithium aluminum hydride in 52 parts of 1,2-dimethoxyethane under argon atmosphere were added 1.39 parts of intermediate 4 during 5 minutes. The reaction mixture was stirred first for 2 hours at 0°C and then for 72 hours at reflux temperature. After cooling, the reaction mixture was quenched with a solution of 1.3 parts of water and 3.6 parts of tetrahydofuran, 1.3 parts of a sodium hydroxide solution 15% and 3.9 parts of water. After stirring for 1 hour, the whole was filtered. The residue was refluxed for 5 minutes in 45 parts of tetrahydrofuran and then refiltered. The combined filtrates were dried, filtered and evaporated under reduced pressure, yielding 1.24 parts (100%) of 2,3,4,5-tetrahydro-3-methyl-4-propyl-1H-1,4-benzodiazepin-9-amine (interm. 5).

Example 2

a) To a stirred and cooled (-12°C) mixture of 9.10 parts of 2-amino-3-nitrobenzoic acid, 6.95 parts of methyl L-α-alanine monohydrochloride, 13.50 parts of 1-hydroxy-1H-1,2,4-benzotriazole monohydrate and 178 parts of tetrahydrofuran were added portion-wise 5.05 parts of N-methylmorpholine and, after 5 minutes, 10.3 parts of N,N′-methanetetraylbis[cyclohexanamine] under argon atmosphere. Stirring was continued for 5 1/2 hours at -12°C and for 15 hours at room temperature. After cooling to 0°C for 1/2 hour, the reaction mixture was filtered. The filtrate was evaporated and the residue was partitioned between ethyl acetate and $NaHCO_3$ (sat). The ethyl acetate layer was separated, washed with $NaHCO_3$ (sat.), dried, filtered and evaporated. The residue was triturated with hexane, filtered and dried, yielding 13.08 parts (97.9%) of (-)-methyl (S)-2-[(2-amino-3-nitrobenzoyl)amino]propanoate; mp. 132.9°C (interm. 6).

b) A mixture of 12.58 parts of intermediate 6, 3.50 parts of palladium-on-charcoal catalyst 10% and 158 parts of ethanol was hydrogenated in a Parr apparatus for 4 hours at room temperature and a pressure of 3.1 $10^5$ Pa. The catalyst was filtered off over diatomaceous earth and the filtrate was evaporated. The oily residue was placed under vacuum (3.3 $10^3$ Pa.) and was heated at 150°C for 10 minutes and at 202°C for 40 minutes while stirring. After cooling, the crushed solid was triturated with ethanol. The product was filtered off and washed with cold ethanol and 1,1′-oxybisethane, yielding 5.58 parts (57.7%) of (+)-(S)-9-amino-3,4-dihydro-3-methyl-1H-1,4-benzodiazepine-2,5-dione (interm. 7).

c) At 25°C and under an argon stream, 5.00 parts of intermediate 7 were added to a suspension of 5.55 parts of lithium aluminum hydride in 154.5 parts of 1,4-dioxane. The reaction mixture was refluxed for 5 hours. After cooling to 10°C, 5.55 parts of water, 9.16 parts of NaOH 15% and 16.65 parts of water were added successively. The whole was stirred for 2 hours and then filtered. The precipitate was washed successively with 178 parts of hot tetrahydrofuran and 133 parts of hot dichloromethane. The combined filtrates were dried, filtered and evaporated. The residue was poured into a solution of 7.36 parts of N-methylmorpholine in 133 parts of dichloromethane. The whole was added to a solution of 4.82 parts of trichloromethyl chloroformate in 160 parts of dichloromethane over a period of 15 minutes at 0°C and under an argon stream. After stirring for 10 minutes at 0°C, the reaction mixture was warmed to room temperature and concentrated by evaporation. 70 Parts of an aqueous 1,4-dioxane solution (15%) were added to the residue and the mixture was heated on a steam-bath under a nitrogen stream for 45 minutes, cooled and extracted with dichloromethane (2x66.5 parts). The aqueous layer was filtered and basified with $NH_4OH$

(conc.). The precipitate was filtered off, washed with a small amount of cold water, dried and triturated twice with 6.24 parts of 2-propanol, yielding 1.59 parts (32.1%) of (+)-(S)-4,5,6,7-tetrahydro-5-methyl-imidazo[4,5,1-jk] [1,4]benzodiazepin-2(1H)-one; mp. 206.5°C (interm. 8).

d) To a stirred mixture of 0.64 parts of intermediate 8, 0.5 parts of sodium carbonate, 0.52 parts of potassium iodide and 9.4 parts of N,N-dimethylformamide were added 0.56 parts of 1-bromo-3-methyl-2-butene at room temperature under argon atmosphere. After stirring for 24 hours, the solvent was removed under reduced pressure. The residue was partitioned between 32 parts of dichloromethane and 35 parts of a sodium chloride solution. The aqueous phase was extracted again with 32 parts of dichloromethane. The combined organic layers were washed with 35 parts of a sodium chloride solution, dried, filtered and evaporated under vacuo. The residue was crystallized from 6.5 parts of acetonitrile. The crystallized product was filtered off and dried for 16 h at 81°C under high vacuum, yielding 0.38 parts (45 %) of (+)-(S)-4,5,6,7-tetrahydro-5-methyl-6-(3-methyl-2-butenyl)imidazo [4,5,1-jk]-[1,4]benzodiazepin-2(1H)-one; mp. 136.4 °C (interm. 9).

e) A suspension of 38.16 parts of intermediate 9 and 15 parts of sodium carbonate in 578 parts of phosphoryl chloride was stirred for 2 days at 60°C under a nitrogen atmosphere. The excess of phosphoryl chloride was distilled off under vacuum at 30-50°C. The resulting solid was cooled (ice-bath) and then dissolved in 500 parts of water. While swirling vigorously, the solution was basified by the slow addition of 1000 ml. of $NaHCO_3$ (sat.). The product was extracted with dichloromethane (3x355 parts) and the combined extracts were washed with $NaHCO_3$ (sat.) and NaCl (sat.), dried, filtered and evaporated, yielding 27 parts (66.5%) of (S)-2-chloro-4,5,6,7-tetrahydro-5-methyl-6-(3-methyl-2-butenyl)-imidazo[4,5,1-jk] [1,4]benzodiazepine (interm. 10).

Example 3

a) A solution of 2.6 parts of methyl 2-bromo-3-nitrobenzoate, 1.75 parts of N-[(2-amino-1-methyl)ethyl]benzenemethanamine and 1.06 parts of sodium carbonate in 8 parts of 1-butanol was stirred and refluxed for 30 minutes. The solvent was evaporated. To the residue were added 20 parts of water and the product was extracted twice with 30 parts of trichloromethane. The combined extracts were dried, filtered and evaporated. From the oily free base, the hydrochloride salt was prepared in the usual manner. The salt was filtered off, washed with 2-propanol and dried, yielding 3.4 parts (89.5%) of methyl 3-nitro-2-[[2-methyl-2-[(phenylmethyl)amino]ethyl]amino]benzoate hydrochloride; mp. 204°C (interm. 11).

b) A mixture of 3.8 parts of intermediate 11, 15 parts of a sodium hydroxide solution 2 N and 4 parts of 2-propanol was stirred and refluxed for one hour. To the boiling reaction mixture there was then added a solution of 3 parts of concentrated hydrochloric acid and 5 parts of water. After cooling, the product was filtered off, washed with water and recrystallized from 80 parts of glacial acetic acid, yielding 3 parts (82%) of 3-nitro-2-[[[2-[(phenylmethyl)amino]-2-methyl]ethyl]amino]benzoic acid; mp. 227°C (interm. 12).

c) A mixture of 189.3 parts of intermediate 12, 400 parts of thionyl chloride and 400 parts of methylbenzene was stirred and refluxed for 2 hours. The solvent was evaporated and the residue was taken up in 600 parts of methylbenzene. The whole was treated with a sodium hydrogen carbonate solution. The separated organic layer was dried on anhydrous sodium carbonate, filtered and concentrated to a volume of about 500 parts. After standing at room temperature, the product partly precipitated. It was filtered off (the filtrate was set aside), washed successively with 2-propanol and 1,1′-oxybisethane and dried, yielding a first fraction of 123.5 parts of crude 2,3,4,5-tetrahydro-3-methyl-9-nitro-4-(phenylmethyl)-1H-1,4-benzodiazepin-5-one. From the mother liquor, the solvent was evaporated. The residue was dissolved in 160 parts of boiling 2-propanol and crystallized at room temperature. The precipitated product was filtered off, washed successively with 2-propanol and 1,1′-oxybisethane and dried, yielding a second less pure fraction of 28 parts of 2,3,4,5-tetrahydro-3-methyl-9-nitro-4-(phenylmethyl)-1H-1,4-benzodiazepin-5-one. Both crude fractions were recrystallized from ethanol, yielding 137 parts (85%) of 2,3,4,5-tetrahydro-3-methyl-9-nitro-4-(phenylmethyl)-1H-1,4-benzodiazepin-5-one; mp. 125°C (interm. 13).

d) To a stirred and refluxing suspension of 14 parts of lithium aluminum hydride in 40 parts of benzene and 50 parts of tetrahydrofuran was added a solution of 20.2 parts of intermediate 13 in 200 parts of tetrahydrofuran and the whole was further stirred and refluxed for 2.5 hours. The reaction mixture was cooled in crushed ice and decomposed by successive additions of water, sodium hydroxide solution 15% and again with water. The inorganic material was filtered off and the filtrate was evaporated. To the residue were added 40 parts of methylbenzene and this solution was evaporated to dryness, yielding 19.8 parts (87.6%) of 9-amino-2,3,4,5-tetrahydro-3-methyl-4-(phenylmethyl)-1H-1,4-benzodiazepin-5-one as a red-coloured, oily residue (interm. 14) which was used without further purification for the preparation of the next step.

e) A mixture of 19.8 parts of intermediate 14 and 7.2 parts of urea was heated to a temperature between 210-220°C until foaming and evolution of gaseous ammonia ceased (about 10 minutes). The reaction was cooled to about 100°C and boiled with 120 parts of hydrochloric acid solution 1 N. The solution was decanted from the oily residue, treated with activated charcoal and filtered. The filtrate was cooled, alkalized with ammonium hydroxide and the product was extracted once with 75 parts of trichloromethane and once with 150 parts of trichloromethane. The combined extracts were dried and evaporated. The residue was triturated in 24 parts of 2-propanol, filtered off and recrystallized from ethanol and then from 4-methyl-2-pentanone, yielding 2.5 parts (11.5%) of 4,5,6,7-tetrahydro-5-methyl-6-(phenylmethyl)imidazo-[4,5,1-jk][1,4]benzodiazepin-2(1H)-one; mp. 205°C (interm. 15).

f) A mixture of 8 parts of intermediate 15, 1 part of palladium-on-charcoal catalyst 10% in 80 parts of glacial acetic acid was hydrogenated at about 38°C. After the calculated amount of hydrogen was taken up, the catalyst was filtered off and the acetic acid was evaporated. The residue was dissolved in 75 parts of water and the solution was alkalized with 30 parts of concentrated ammonium hydroxide solution. The product crystallized at room temperature. It was filtered off, washed with water and recrystallized from 20 parts of 2-propanol, yielding 3.7 parts (66.8%) of 4,5,6,7-tetrahydro-5-methyl-imidazo-[4,5,1-jk][1,4]benzodiazepin-2(1H)-one; mp. 190.5°C (interm. 16).

g) To a stirred solution of 1.0 part of intermediate 16, 0.816 parts of potassium iodide and 0.782 parts of sodium carbonate in 56.4 parts of N,N-dimethylformamide was added dropwise a solution of 0.88 parts of 1-bromo-3-methyl-2-butene in 14 parts of N,N-dimethylformamide. After stirring for 22.5 hours at room temperature, the reaction mixture was concentrated in vacuo at ~70°C. The residue was partitioned twice between 130 parts of dichloromethane and 100 parts of a mixture of water and a saturated aqueous sodium hydrogen carbonate solution (50:50 by volume). The combined aqueous layers were extracted with 78 parts of dichloromethane. The dichloromethane layers were combined and extracted with 100 parts of a saturated sodium chloride solution. The extract was dried, filtered and concentrated in vacuo at ~40°C. The residue was crystallized twice from 16 parts of acetonitrile. The whole was cooled for 45 minutes at 0-5°C; the crystallized product was filtered off, washed with 4 parts cold (0-5°C) acetonitrile and dried overnight in vacuo at 78°C, yielding 0.805 parts (60.3%) of (±)-4,5,6,7-tetrahydro-5-methyl-6-(3-methyl-2-butenyl)imidazo[4,5,1-jk][1,4]benzodiazepin-2(1H)-one; mp. 158.0°C (interm. 17).

h) A suspension of 1.0 part of intermediate 17 in 8.25 parts of phosphoryl chloride was heated for 15 hours at 90°C under a nitrogen atmosphere. The reaction mixture was evaporated and the residue was partitioned between $NaHCO_3$ (sat.) and dichloro methane. The aqueous layer was re-extracted with dichloromethane. The combined organic layers were washed with $NaHCO_3$ (sat.) and NaCl (sat.), dried, filtered and evaporated, yielding 1.05 parts (98.3%) of 2-chloro-4,5,6,7-tetrahydro-5-methyl-6-(3-methyl-2-butenyl)imidazo[4,5,1-jk] [1,4]benzodiazepine (interm. 18).

## Example 4

a) A mixture of 41.49 parts of 6-chloro-2H-3,1-benzoxazine-2,4(1H)-dione and 31.40 parts of methyl L-α-alanine monohydrochloride in 108 parts of pyridine was refluxed for 10 hours under an argon atmosphere. The reaction mixture was cooled and stirred at room temperature for 12 hours. The precipitate was filtered off, rinsed with water and triturated in ethanol. The product was filtered off and rinsed with ethanol, yielding 24.77 parts (52.5%) of (S)-7-chloro-3,4-dihydro-3-methyl-1H-1,4-benzodiazepine-2,5-dione (interm. 19).

b) 24.55 Parts of intermediate 19 were added portionwise to 142 parts of nitric acid at 0°C and under an argon atmosphere. After 3 1/2 hours at 0°C, the solution was slowly added to 450 parts of ice while stirring. The precipitate was filtered off, rinsed with water and dried at room temperature overnight, yielding 27.84 parts (93.9%) of (S)-7-chloro-3,4-dihydro-3-methyl-9-nitro-1H-1,4-benzodiazepine-2,5-dione (interm. 20).

c) To a cooled (0°C) suspension of 18.2 parts of lithium aluminum hydride in 261 parts of 1,2-dimethoxyethane were added portionwise 16.14 parts of intermediate 20 under a nitrogen atmosphere. The mixture was stirred for 2 hours at 0°C and for 40 hours at reflux temperature. After cooling to 0°C, there were added a mixture of 18.2 parts of water and 48.1 parts of tetrahydrofuran, 21.1 parts of NaOH 15% and 54.6 parts of water. The mixture was stirred for 1 hour at room temperature and was then filtered. The precipitate was refluxed in tetrahydrofuran for 5 min. and filtered off again. The combined filtrates were dried, filtered and evaporated and the residue was dissolved in 399 parts of dichloromethane. After drying and filtering, this solution was combined with 18.2 parts of N-methylmorpholine and the whole was added dropwise to a mixture of 11.9 parts of trichloromethyl chloroformate and 665 parts of dichloromethane at 0°C and under argon. The whole was evaporated and the residue was taken up in 150 ml of a mixture of water and 1,4-dioxane 85:15. The mixture was heated for 2 hours on a steam-bath under nitrogen. After cooling, the solid was filtered off and dissolved in 80 parts of water. The solution was basified with $NH_4OH$ and stirred for

45 min. The product was filtered off and crystallized successively from acetonitrile and 2-propanol, yielding 2.28 parts (16%) of (+)-(S)-9-chloro-4,5,6,7-tetrahydro-5-methylimidazo[4,5,1-jk] [1,4]benzodiazepin-2(1 H)-one; mp. 202.2°C; $[\alpha]_D^{20}$ = +72.6° (c = 0.98% in methanol) (interm. 21).

d) To a stirred mixture of 2.99 parts of intermediate 21, 2.00 parts of sodium carbonate, 2.08 parts of potassium iodide and 37.6 parts of N,N-dimethylformamide were added 2.24 parts of 1-bromo-3-methyl-2-butene under an argon atmosphere. After stirring for 4 days at room temperature, the reaction mixture was evaporated and the residue was partitioned between water and dichloromethane. The organic layer was washed with NaCl (sat.), dried, filtered and evaporated. The residue was crystallized from acetonitrile (2x). The product was filtered off, washed with cold acetonitrile and dried, yielding 1.74 parts (45.2%) of (+)-(S)-9-chloro-4,5,6,7-tetrahydro-5-methyl-6-(3-methyl-2-butenyl)imidazo[4,5,1-jk] [1,4]benzodiazepin-2(1H)-one; mp. 135.6°C (interm. 22).

e) A suspension of 2.5 parts of intermediate 22 and 0.87 parts of sodium carbonate in 33 parts of phosphoryl chloride was stirred for 24 hours at 90°C under a nitrogen atmosphere. The excess of phosphoryl chloride was distilled off under vacuum. The resulting solid was cooled (ice-bath) and then taken up in water. While swirling vigorously, the mixture was basified by the slow addition of $NaHCO_3$ (sat.). The product was extracted with dichloromethane (3x44.3 parts) and the combined extracts were washed with $NaHCO_3$ (sat.) and NaCl (sat.), dried, filtered and evaporated, yielding 2.57 parts (97.0%) of (S)-2,9-dichloro-4,5,6,7-tetrahydro-5-methyl-6-(3-methyl-2-butenyl)imidazo[4,5,1-jk] [1,4]benzodiazepine (interm. 23).

f) A mixture of 298.42 parts of intermediate 20 and 3324 parts of ethanol was hydrogenated at 50°C and normal pressure with 21.04 parts of platinum-on-charcoal catalyst 5%. At the end of the hydrogenation, the temperature was raised to 70°C. The reaction mixture was filtered while hot and the catalyst was washed with boiling ethanol. The filtrate was stirred overnight in an ice-bath and was then concentrated. The residue was cooled on ice. The precipitate was filtered off, washed with methylbenzene and dried in vacuo at 50°C, yielding 187.7 parts (74.6%) of (S)-9-amino-7-chloro-3,4-dihydro-3-methyl-1H-1,4-benzodiazepine-2,5-dione (int. 24).

g) To a cooled (ice-bath) suspension of 29.3 parts of lithium aluminum hydride in 392 parts of 1,2-dimethoxyethane were added portionwise 30.78 parts of intermediate 24 under a nitrogen atmosphere. The mixture was refluxed for 22 hours, cooled to 0-5°C and then worked up with a mixture of 36.5 parts of 1,2-dimethoxyethane and 42 parts of water. Next there were added 48.7 parts of NaOH 15% and 135 parts of water. After stirring for 15 min., the whole was filtered and the precipitate was washed with 1,2-dimethoxyethane. The combined filtrates were evaporated and the residue was dried, yielding 25.4 parts (93.7%) of (S)-7-chloro-2,3,4,5-tetrahydro-3-methyl-1H-1,4-benzodiazepin-9-amine (int. 25).

h) To a heated (40°C) solution of 91 parts of intermediate 25 in 500 ml of 1,2-dimethoxyethane were successively added 1253 parts of N,N-dimethylformamide, 66.98 parts of sodium carbonate and 71.38 parts of potassium iodide. After cooling to 0-5°C, there was added dropwise a solution of 271.3 parts of 1-chloro-3-methyl-2-butene in 270 parts of N,N-dimethylformamide under a nitrogen atmosphere. The whole was stirred for 18 hours at 0-5°C and was then partitioned between dichloromethane and water. The aqueous layer was separated and re-extracted with dichloromethane. The combined dichloromethane layers were washed with water (7x), dried, filtered and evaporated. The residue was purified by column chromatography (silica gel; $C_6H_5CH_3$/i.$C_3H_7OH$ 98:2). The eluent of the desired fraction was evaporated, yielding 43.64 parts (51.8%) of (S)-7-chloro-2,3,4,5-tetrahydro-3-methyl-4-(3-methyl-2-butenyl)-1H-1,4-benzodiazepin-9-amine (int. 26).

## B. Preparation of the final compounds

### Example 5

To a stirred solution of 1.24 parts of intermediate 5 in 4.5 parts of ethanol and 1.1 parts of water were added 0.36 parts of potassium hydroxide. After stirring for 8 minutes at room temperature, 0.5 parts of carbon disulfide were added. The reaction mixture was stirred for 10 minutes and then heated for 1 hour in an oil bath at 90°C. After cooling to room temperature, the mixture was diluted with 5.6 parts of water and then 0.47 parts of acetic acid were added. The mixture was filtered and the filtrate was treated with concentrated ammonium hydroxide. The whole was extracted twice with 32.5 parts of dichloromethane. The combined extracts were dried, filtered and evaporated under reduced pressure. The residue was purified by column chromatography (silica gel; $CH_2Cl_2/CH_3OH$ 10:1). The pure fractions were collected and the eluent was evaporated. The residue was triturated in acetonitrile. The product was filtered off and dried, yielding 0.30 parts (20.4%) of 4,5,6,7-tetrahydro-5-methyl-6-propylimidazo-[4.5.1-jk][1,4]benzodiazepine-2(1H)-thione; mp. 149-151°C (compound 1). Following the same procedure and starting from 2,3,4,5-tetrahydro-3-methyl-4-allyl-1H-1,4-benzodiazepin-9-amine, there can also be prepared 4,5,6,7-tetrahydro-5-methyl-6-allylimidazo-[4.5.1-jk] [1,4]benzodiazepine-2(1H)-thione (compound 2).

Example 6

To a solution of 2.57 parts of intermediate 23 in 27.7 parts of ethanol were added 1.21 parts of thiourea. After refluxing for 24 hours, the reaction mixture was evaporated and the residue was partitioned between $NaHCO_3$ (sat.) and dichloromethane. The organic layer was washed with $NaHCO_3$ (sat.), water and NaCl (sat.), dried, filtered and evaporated. The residue was purified twice by column chromatography (flash; silica gel; $CH_2Cl_2/CH_3OH$ 30:1 ; HPLC; silica gel ; $CH_3COOC_2H_5$/hexane 4:6). The eluent of the desired fraction was evaporated, yielding 0.34 parts (13.3%) of (+)-(S)-9-chloro-4,5,6,7-tetrahydro-5-methyl-6-(3-methyl-2-butenyl)imidazo[4,5,1-jk][1,4]benzodiazepine-2(1H)-thione; mp. 180.3°C; $[\alpha]_D^{20}$ = +8.3° (c = 0.96% in methanol) (compound 3).

In a similar manner there were also prepared

(±)-4,5,6,7-tetrahydro-5-methyl-6-(3-methyl-2-butenyl)imidazo[4,5,1-jk][1,4]benzodiazepine-2(1H)-thione; mp. 128.0°C (dec.) (compound 4).

(+)-(S)-4,5,6,7-tetrahydro-5-methyl-6-(3-methyl-2-butenyl)imidazo[4,5,1-jk] [1,4]benzodiazepine-2(1H)-thione; mp. 174.5°C; $[\alpha]_D^{20}$ = +15.95° (c = 1% in ethanol) (compound 5).

Example 7

A mixture of 43.0 parts of intermediate 26,3152 parts of dichloromethane and 30.1 parts of N,N-diethylethanamine was stirred at 0-5° under a nitrogen atmosphere and screened from light. A solution of 16.3 parts of thiophosgene in 299 parts of dichloromethane was added dropwise at 0-5°C. The whole was stirred for 1 hour at 0-5°C and was then concentrated to about 1000 ml. The residue was washed with water (2x), dried, filtered and evaporated. The residue was purified by column chromatography (silica gel ; $C_6H_5CH_3/CH_3COOC_2H_5$ 88:12). The eluent of the desired fraction was evaporated, yielding 19.5 parts (51.2%) of (+)-(S)-9-chloro-4,5,6,7-tetrahydro-5-methyl-6-(3-methyl-2-butenyl)imidazo[4,5,1-jk] [1,4]benzodiazepine-2(1H)-thione; mp. 186.3°C; $[\alpha]_D^{20}$ = +11.79° (conc. = 1% in $CH_3OH$) (compound 3).

All other compounds listed in table I may be prepared following the procedure of the example referred to in the column Ex. No.

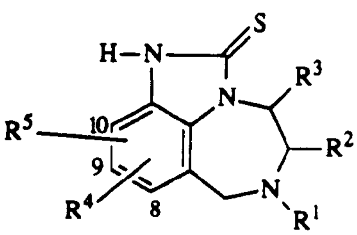

| Comp. No. | Ex. No | $R^1$ | $R^2$ | $R^3$ | $R^4$, $R^5$ | physical data |
|---|---|---|---|---|---|---|
| 1 | 5 | $CH_2$-$CH_2$-$CH_3$ | $CH_3$ | H | H | mp. 149-151°C |
| 2 | 5 | $CH_2$-CH=$CH_3$ | $CH_3$ | H | H | |
| 3 | 6 | $CH_2$-CH=C($CH_3$)$_2$ | $CH_3$ | H | 9-Cl | (S);mp. 186.3°C / $[\alpha]_D^{20}$ = +11.79° (c = 1% in methanol) |
| 4 | 6 | $CH_2$-CH=C($CH_3$)$_2$ | $CH_3$ | H | H | mp. 128.0°C (dec.) |
| 5 | 6 | $CH_2$-CH=C($CH_3$)$_2$ | $CH_3$ | H | H | (S);mp. 174.5°C / $[\alpha]_D^{20}$ = +15.95° (c= 1% in ethanol) |
| 6 | 6 | $CH_2$-CH=C($CH_3$)$_2$ | $CH_3$ | H | H | (R);mp. 178.5°C / $[\alpha]_D^{20}$ = -16.43° (c = 0.1% in ethanol) |
| 7 | 6 | $CH_2$-CH=C($CH_3$)$_2$ | $CH_3$ | H | 9-Cl | (S)/HCl;mp. >270°C |
| 8 | 6 | $CH_2$-C($CH_3$)=$CH_2$ | $CH_3$ | H | H | (S);mp. 137.6°C / $[\alpha]_D^{25}$ = +26.2° (c=1.09 % in methanol) |
| 9 | 6 | $CH_2$-c.$C_3H_5$ | $CH_3$ | H | H | (S);mp. 197.4°C / $[\alpha]_D^{25}$ = +21.4° (c = 0.83 % in $CHCl_3$) |
| 10 | 6 | $CH_2$-CH=C($CH_3$)$_2$ | $CH_3$ | H | 9-F | (S); mp. 168.5-171°C |
| 11 | 6 | $CH_2$-CH=C($CH_3$)$_2$ | $CH_3$ | H | 9-$CH_3$ | (S); |
| 12 | 6 | $CH_2$-CH=C($CH_3$)$_2$ | $CH_3$ | H | 9-OH | (S); |
| 13 | 6 | $CH_2$-CH=C($CH_3$)$_2$ | $CH_3$ | H | 9-CN | (S); |
| 14 | 6 | $CH_2$-CH=C($CH_3$)$_2$ | $CH_3$ | H | 9-$NO_2$ | (S); |
| 15 | 6 | $CH_2$-CH=C($CH_3$)$_2$ | $CH_3$ | H | 8-$CH_3$ | (S); mp. 147.6° / $[\alpha]_D^{25}$ = +7.4° (c = 0.33 % in methanol) |
| 16 | 6 | $CH_2$-CH=C($CH_3$)$_2$ | $CH_3$ | H | 9-$CF_3$ | (S); |
| 17 | 6 | $CH_2$-CH=C($CH_3$)$_2$ | $CH_3$ | H | 9-Cl,10-Cl | (S);mp. 168-172°C |
| 18 | 6 | $CH_2$-CH=C($CH_3$)$_2$ | $CH_3$ | H | 9-$OCH_3$, 10-$OCH_3$ | (S); |
| 19 | 6 | $CH_2$-CH=C($CH_3$)$_2$ | $CH_3$ | H | 8-$CH_3$,9-Cl | (S); |
| 20 | 5 | $CH_2$-c.$C_3H_5$ | $CH_3$ | H | 9-Cl | (S); mp. 171-174°C |
| 21 | 6 | $CH_2$-CH=C($CH_3$)$_2$ | CH($CH_3$)$_2$ | H | H | (S); |
| 22 | 6 | $CH_2$-CH=C($CH_3$)$_2$ | CH($CH_3$)$_2$ | H | 9-Cl | (S); |
| 23 | 6 | $CH_2$-CH=C($CH_3$)$_2$ | H | $CH_3$ | H | (S); |
| 24 | 6 | $CH_2$-CH=C($CH_3$)$_2$ | $CH_3$ | $CH_3$ | H | trans; 156°C |
| 25 | 6 | $CH_2$-CH=C($CH_3$)$_2$ | $CH_3$ | $CH_3$ | H | cis |

| Comp. No. | Ex. No | $R^1$ | $R^2$ | $R^3$ | $R^4$, $R^5$ | physical data |
|---|---|---|---|---|---|---|
| 26 | 6 | $CH_2$-CH=C($CH_3$)$_2$ | H | H | 9-Cl | |
| 27 | 6 | $CH_2$-CH=C($CH_3$)$_2$ | $CH_3$ | H | 9-O$CH_3$ | (S); |
| 28 | 6 | $CH_2$-CH=C($CH_3$)$_2$ | $CH_3$ | H | 9-Cl | (R); mp. 183-184°C |
| 29 | 6 | $CH_2$-CH=C($CH_3$)$_2$ | H | $CH_3$ | 9-Cl | rac. |
| 30 | 6 | $CH_2$-CH=C($CH_3$)$_2$ | H | $CH_3$ | H | rac. |

## C. Pharmacological example

### Example 8

A rapid, sensitive and automated assay procedure was used for the in-vitro evaluation of anti-HIV agents. An HIV-1 transformed T4-cell line, MT-4, which was previously shown (Koyanagi et al., Int. J. Cancer, 36, 445-451, 1985) to be highly susceptible to and permissive for HIV infection, served as the target cell line. Inhibition of the HIV-induced cytopathic effect was used as the end point. The viability of both HIV- and mock-infected cells was assessed spectrophotometrically via the in-situ reduction of 3-(4,5-dimethylthiazol-2-yl)-2,5-diphenyltetrazolium bromide (MTT). The 50% cytotoxic dose ($CD_{50}$ in µg/ml) was defined as the concentration of compound that reduced the absorbance of the mock-infected control sample by 50%. The percent protection achieved by the compound in HIV-infected cells was calculated by the following formula:

$$\frac{(OD_T)_{HIV} - (OD_C)_{HIV}}{(OD_C)_{MOCK} - (OD_C)_{HIV}} \text{ expressed in \%,}$$

whereby $(OD_T)_{HIV}$ is the optical density measured with a given concentration of the test compound in HIV-infected cells; $(OD_C)_{HIV}$ is the optical density measured for the control untreated HIV-infected cells; $(OD_C)_{MOCK}$ is the optical density measured for the control untreated mock-infected cells; all optical density values were determined at 540 nm. The dose achieving 50% protection according to the above formula was defined as the 50% effective dose ($ED_{50}$ in µg/ml). The ratio of $CD_{50}$ to $ED_{50}$ was defined as the selectivity index (SI).

Table 2 : 50% cytotoxic ($CD_{50}$), 50% effective dose ($ED_{50}$) and selectivity index (SI).

| compound | $CD_{50}$ (µg/ml) | $ED_{50}$ (µg/ml) | SI |
|---|---|---|---|
| 1 | 147 | 0.155 | 948 |
| 3 | 10 | 0.0005 | 20400 |
| 4 | 25 | 0.013 | 1923 |
| 5 | 325 | 0.008 | 40625 |
| 6 | ≥250 | 0.045 | ≥5555 |
| 7 | 23 | 0.0018 | 12778 |
| 8 | 4.6 | 0.0126 | 365 |
| 9 | >250 | 0.0166 | >15029 |

## D. Composition Examples

### Example 9: ORAL DROPS

500 Parts of the A.I. was dissolved in 0.5 l of 2-hydroxypropanoic acid and 1.5 l of the polyethylene glycol

at 60~80°C. After cooling to 30~40°C there were added 35 l of polyethylene glycol and the mixture was stirred well. Then there was added a solution of 1750 parts of sodium saccharin in 2.5 l of purified water and while stirring there were added 2.5 l of cocoa flavor and polyethylene glycol q.s. to a volume of 50 l, providing an oral drop solution comprising 10 mg/ml of A.I.. The resulting solution was filled into suitable containers.

Example 10 : ORAL SOLUTION

9 Parts of methyl 4-hydroxybenzoate and 1 part of propyl 4-hydroxybenzoate were dissolved in 4 l of boiling purified water. In 3 l of this solution were dissolved first 10 parts of 2,3-dihydroxybutanedioic acid and thereafter 20 parts of the A.I. The latter solution was combined with the remaining part of the former solution and 12 l 1,2,3-propanetriol and 3 l of sorbitol 70% solution were added thereto. 40 Parts of sodium saccharin were dissolved in 0.5 l of water and 2 ml of raspberry and 2 ml of gooseberry essence were added. The latter solution was combined with the former, water was added q.s. to a volume of 20 l providing an oral solution comprising 5 mg of the active ingredient per teaspoonful (5 ml). The resulting solution was filled in suitable containers.

Example 11 : CAPSULES

20 Parts of the A.I., 6 parts sodium lauryl sulfate, 56 parts starch, 56 parts lactose, 0.8 parts colloidal silicon dioxide, and 1.2 parts magnesium stearate were vigorously stirred together. The resulting mixture was subsequently filled into 1000 suitable hardened gelatin capsules, comprising each 20 mg of the active ingredient.

Example 12: FILM-COATED TABLETS

Preparation of tablet core

A mixture of 100 parts of the A.I., 570 parts lactose and 200 parts starch was mixed well and thereafter humidified with a solution of 5 parts sodium dodecyl sulfate and 10 parts polyvinylpyrrolidone (Kollidon-K 90 ®) in about 200 ml of water. The wet powder mixture was sieved, dried and sieved again. Then there was added 100 parts microcrystalline cellulose (Avicel ®) and 15 parts hydrogenated vegetable oil (Sterotex ®). The whole was mixed well and compressed into tablets, giving 10.000 tablets, each containing 10 mg of the active ingredient.

Coating

To a solution of 10 parts methyl cellulose (Methocel 60 HG®) in 75 ml of denaturated ethanol there was added a solution of 5 parts of ethyl cellulose (Ethocel 22 cps ®) in 150 ml of dichloromethane. Then there were added 75 ml of dichloromethane and 2.5 ml 1,2,3-propanetriol. 10 Parts of polyethylene glycol was molten and dissolved in 75 ml of dichloromethane. The latter solution was added to the former and then there were added 2.5 parts of magnesium octadecanoate, 5 parts of polyvinylpyrrolidone and 30 ml of concentrated colour suspension (Opaspray K-1-2109®) and the whole was homogenated. The tablet cores were coated with the thus obtained mixture in a coating apparatus.

Example 13: INJECTABLE SOLUTION

1.8 Parts methyl 4-hydroxybenzoate and 0.2 parts propyl 4-hydroxybenzoate were dissolved in about 0.5 l of boiling water for injection. After cooling to about 50°C there were added while stirring 4 parts lactic acid, 0.05 parts propylene glycol and 4 parts of the A.I.. The solution was cooled to room temperature and supplemented with water for injection q.s. ad 1 l, giving a solution comprising 4 mg/ml of A.I.. The solution was sterilized by filtration (U.S.P. XVII p. 811) and filled in sterile containers.

Example 14: SUPPOSITORIES

3 Parts A.I. was dissolved in a solution of 3 parts 2,3-dihydroxybutanedioic acid in 25 ml polyethylene glycol 400. 12 Parts surfactant (SPAN®) and triglycerides (Witepsol 555 ®) q.s. ad 300 parts were molten together. The latter mixture was mixed well with the former solution. The thus obtained mixture was poured into moulds at a temperature of 37-38°C to form 100 suppositories each containing 30 mg/ml of the A.I.

Example 15 : INJECTABLE SOLUTION

60 Parts of A.I. and 12 parts of benzylalcohol were mixed well and sesame oil was added q.s. ad 1 l, giving a solution comprising 60 mg/ml of A.I. The solution was sterilized and filled in sterile containers.

## Claims

1. A compound having the formula

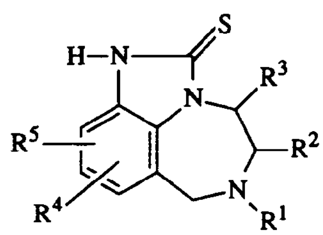

(I),

a pharmaceutically acceptable acid addition salt or a stereochemically isomeric form thereof, wherein

$R^1$ is $C_{1-6}$alkyl, $C_{3-6}$alkenyl, $C_{3-6}$alkynyl, $C_{3-6}$cycloalkyl, or $C_{1-6}$alkyl substituted with aryl or with $C_{3-6}$cycloalkyl;

$R^2$ is hydrogen or $C_{1-6}$alkyl;

$R^3$ is hydrogen or $C_{1-6}$alkyl;

$R^4$ and $R^5$ each independently are hydrogen, $C_{1-6}$alkyl, halo, cyano, nitro, trifluoromethyl, hydroxy, $C_{1-6}$alkyloxy, amino or mono-or di($C_{1-6}$alkylamino); and

aryl is phenyl optionally substituted with from 1 to 3 substituents independently selected from $C_{1-6}$alkyl, halo, hydroxy, $C_{1-6}$alkyloxy, amino, nitro and trifluoromethyl.

2. A compound according to claim 1 wherein $R^1$ is $C_{1-6}$alkyl, $C_{3-6}$alkenyl, $C_{3-6}$alkynyl or $C_{1-6}$alkyl substituted with aryl or with $C_{3-6}$cycloalkyl; $R^4$ and $R^5$ each independently are hydrogen, $C_{1-6}$alkyl, halo, cyano, nitro, trifluoromethyl, hydroxy or $C_{1-6}$alkyloxy.

3. A compound according to claim 2 wherein $R^1$ is $C_{3-6}$alkyl, $C_{3-6}$alkenyl, or $C_{1-6}$alkyl substituted with $C_{3-6}$cycloalkyl; $R^2$ is $C_{1-6}$alkyl; $R^5$ is hydrogen.

4. A compound according to claim 3 wherein $R^1$ is $C_{3-6}$alkyl, $C_{3-6}$alkenyl or ($C_{3-6}$cycloalkyl)methyl; $R^2$ is methyl; $R^3$ is hydrogen; $R^4$ is hydrogen, methyl, halo, cyano, nitro or trifluoromethyl.

5. A compound according to claim 4 wherein $R^1$ is propyl, 2-propenyl, 2-butenyl, 2-methyl-2-butenyl, 3-methyl-2-butenyl, 2,3-dimethyl-2-butenyl or cyclopropylmethyl; $R^4$ is hydrogen, methyl or chloro.

6. A compound according to claim 1 wherein the compound is selected from 4,5,6,7-tetrahydro-5-methyl-6-propylimidazo-[4.5.1-jk][1,4]benzodiazepine-2(1H)-thione; (+)-(S)-4,5,6,7-tetrahydro-5-methyl-6-(3-methyl-2-butenyl)imidazo[4,5,1-jk] [1,4]benzodiazepine-2(1H)-thione; (+)-(S)-9-chloro-4,5,6,7-tetrahydro-5-methyl-6-(3-methyl-2-butenyl)imidazo[4,5,1-jk] [1,4]benzodiazepine-2(1H)-thione; the pharmaceutically acceptable acid addition salts thereof, and the stereochemically isomeric forms thereof.

7. A compound having the formula

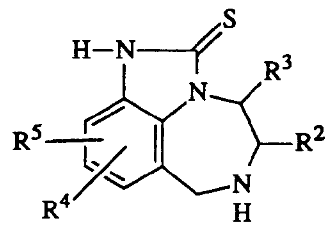

(V)

an acid addition salt or a stereochemically isomeric form thereof, wherein
$R^2$, $R^3$, $R^4$ and $R^5$ are as defined in claim 1.

**8.** A compound having the formula

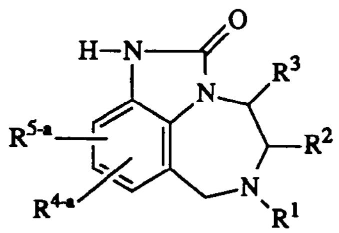

(IV-a)

a pharmaceutically acceptable acid addition salt or a stereochemically isomeric form thereof, wherein $R^1$, $R^2$ and $R^3$ are as defined in claim 1; and

(a) $R^{4-a}$ and $R^{5-a}$ each independently are $C_{1-6}$alkyl, halo, cyano, nitro, trifluoromethyl, hydroxy, $C_{1-6}$alkyloxy, amino- or mono- or di($C_{1-6}$alkyl)amino, or

(b) $R^{4-a}$ is hydrogen and $R^{5-a}$ is cyano, nitro, trifluoromethyl, hydroxy, $C_{1-6}$alkyloxy, amino or mono- or di($C_{1-6}$alkyl)amino.

**9.** A compound as claimed in any one of claims 1 to 6 and claim 8 for use as a medicine.

**10.** A pharmaceutical composition comprising a pharmaceutical carrier and as active ingredient a therapeutically effective amount of a compound as claimed in any one of claims 1 to 6 and claim 8.

**11.** A method of preparing a pharmaceutical composition according to claim 10, characterized in that a therapeutically effective amount of a compound as defined in any of claims 1 to 6 and claim 8 is intimately mixed with a pharmaceutical carrier.

**12.** A process for preparing a compound as claimed in any of claims 1 to 6, characterized by
a) condensing a 9-amino-2,3,4,5-tetrahydro-1H-1,4-benzodiazepine of formula

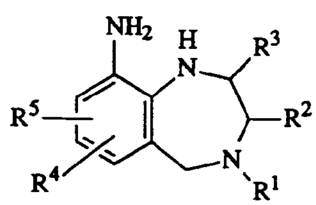

(II)

wherein $R^1$, $R^2$, $R^3$, $R^4$ and $R^5$ are as defined in formula (I) with a reagent of formula L-C(=S)-L (III), wherein L is a leaving group, in a reaction-inert solvent;
b) thionating a 4,5,6,7-tetrahydroimidazo[4,5,1-jk][1,4]-benzodiazepin-2-one of formula

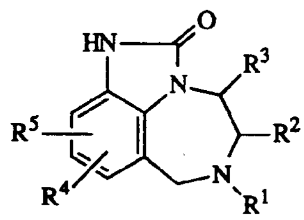

(IV)

wherein $R^1$, $R^2$, $R^3$, $R^4$ and $R^5$ are as defined in formula (I)

i) by reaction with a halogenating reagent and conversion of the thus obtained 2-halo-4,5,6-7-tetrahydroimidazo[4,5,1-jk][1,4]benzodiazepine with thiourea or an alkali metal thiosulfate in a reaction-inert solvent; or

ii) by thionation with 2,4-bis(4-methoxyphenyl)-1,3-dithia-2,4-diphosphetane-2,4-disulfide or phosphorus pentasulfide in an inert solvent;

c) N-alkylating an intermediate of formula

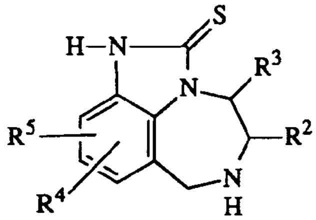

(V)

wherein $R^2$, $R^3$, $R^4$ and $R^5$ are as defined in formula (I) with a reagent of formula $R^1$-W (VI-a) wherein W represents a reactive leaving group and $R^1$ is as defined under formula (I), in a reaction-inert solvent;

d) N-alkylating an intermediate of formula

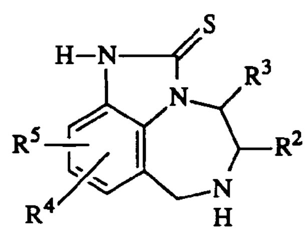

(V)

wherein $R^2$, $R^3$, $R^4$ and $R^5$ are as defined in formula (I) with a ketone or aldehyde of formula $R^{1-b}$=O (VI-b), wherein $R^{1-b}$ represents a geminal bivalent radical derived from $R^{1-a}$-H, $R^{1-a}$ being $C_{1-6}$alkyl, $C_{3-6}$cycloalkyl or $C_{1-6}$alkyl substituted with aryl or with $C_{3-6}$cycloalkyl, wherein two geminal hydrogen atoms are replaced by =O in a reaction-inert solvent, thus yielding a compound of formula

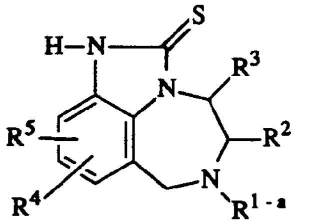

(I-a);

e) thiating a tetrahydroimidazo[4,5,1-jk][1,4]benzodiazepine of formula

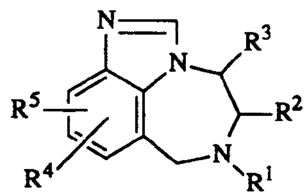

(VII)

wherein $R^1$, $R^2$, $R^3$, $R^4$ and $R^5$ are as defined in formula (I) with elemental sulfur at an elevated temperature;
f) reducing and thiocarbonylating a 9-nitrobenzodiazepine of formula

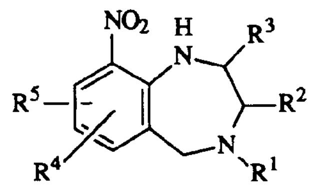

(VIII)

wherein $R^1$, $R^2$, $R^3$, $R^4$ and $R^5$ are as defined in formula (I) in the presence of an alkali metal sulfide or hydrogen sulfide, and carbon disulfide;
g) cyclizing a benzimidazol-2-thione of formula

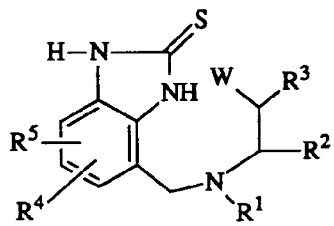

(IX)

wherein $R^1$, $R^2$, $R^3$, $R^4$ and $R^5$ are as defined in formula (I) and W represents a reactive leaving group, in a reaction-inert solvent, and, if desired, converting the compounds of formula (I) into a therapeutically active non-toxic acid addition salt form by treatment with an acid; or conversely, converting the acid salt into the free base with alkali; and/or preparing stereochemically isomeric forms thereof.

**13.** A process for preparing a compound as claimed in claim 7, characterized by
a) condensing a 9-amino-2,3,4,5-tetrahydro-1H-1,4-benzodiazepine of formula

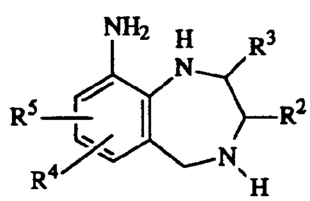

(II-a)

wherein $R^2$, $R^3$, $R^4$ and $R^5$ are as defined in formula (I) with a reagent of formula L-C(=S)-L (III), wherein L is a leaving group, in a reaction-inert solvent;
b) thionating a 4,5,6,7-tetrahydroimidazo[4,5,1-jk] [1,4]-benzodiazepin-2-one of formula

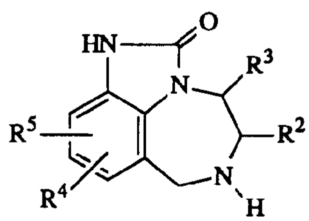

(IV-b)

wherein $R^2$, $R^3$, $R^4$ and $R^5$ are as defined in formula (I)

i) by reaction with a halogenating reagent and conversion of the thus obtained 2-halo-4,5,6-7-tetrahydroimidazo[4,5,1-jk][1,4]benzodiazepine with thiourea or an alkali metal thiosulfate in a reaction-inert solvent; or

ii) by thionation with 2,4-bis(4-methoxyphenyl)-1,3-dithia-2,4-diphosphetane-2,4-disulfide or phosphorus pentasulfide in an inert solvent;

c) thiating a tetrahydroimidazo[4,5,1-jk][1,4]benzodiazepine of formula

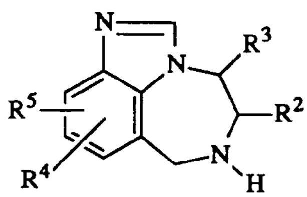

(VII-a)

wherein $R^2$, $R^3$, $R^4$ and $R^5$ are as defined in formula (I) with elemental sulfur at an elevated temperature;

d) reducing and thiocarbonylating a 9-nitrobenzodiazepine of formula

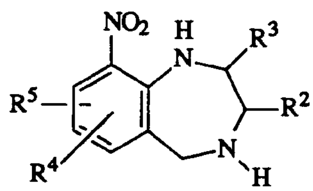

(VIII-a)

wherein $R^2$, $R^3$, $R^4$ and $R^5$ are as defined in formula (I) in the presence of an alkali metal sulfide or hydrogen sulfide, and carbon disulfide;

e) cyclizing a benzimidazol-2-thione of formula

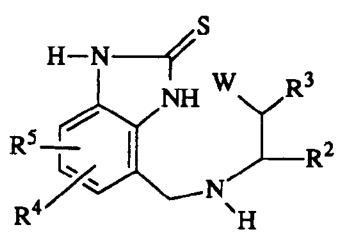

(IX-a)

wherein $R^2$, $R^3$, $R^4$ and $R^5$ are as defined in formula (I) and W represents a reactive leaving group, in a reaction-inert solvent, and, if desired, converting the compounds of formula (I) into a therapeutically active non-toxic acid addition salt form by treatment with an acid; or conversely, converting the acid salt into the free base with alkali; and/or preparing stereochemically isomeric forms thereof.

**14.** A process for preparing a compound as claimed in claim 8, characterized by

a) condensing a 9-amino-2,3,4,5-tetrahydro-1H-1,4-benzodiazepine of formula

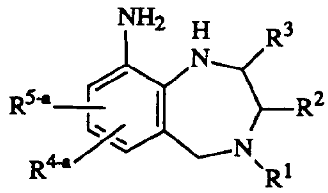

(II-b)

wherein $R^1$, $R^2$, $R^3$, $R^{4-a}$ and $R^{5-a}$ are as defined in claim 8 with a reagent of formula L-C(=O)-L (LVIII), wherein L is a leaving group, in a reaction-inert solvent;
b) N-alkylating an intermediate of formula

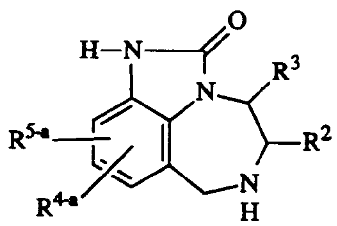

(IV-b-1)

wherein $R^2$, $R^3$, $R^{4-a}$ and $R^{5-a}$ are as defined in claim 8 with a reagent of formula $R^1$-W (VI-a) wherein W represents a reactive leaving group and $R^1$ is as defined under formula (I), in a reaction-inert solvent;
c) N-alkylating an intermediate of formula

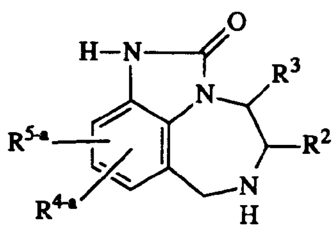

(IV-b-1)

wherein $R^2$, $R^3$, $R^{4-a}$ and $R^{5-a}$ are as defined in claim 8 with a ketone or aldehyde of formula $R^{1-b}$=O (VI-b), wherein $R^{1-b}$ represents a geminal bivalent radical derived from $R^{1-a}$-H, $R^{1-a}$ being $C_{1-6}$alkyl, $C_{3-6}$cycloalkyl or $C_{1-6}$alkyl substituted with aryl or with $C_{3-6}$cycloalkyl, wherein two geminal hydrogen atoms are replaced by =O in a reaction-inert solvent, thus yielding a compound of formula

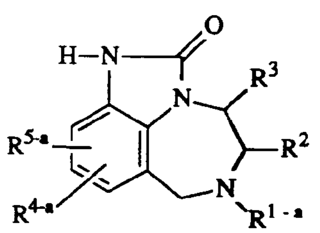

(IV-a-1);

and, if desired, converting the compounds of formula (IV-a) into a therapeutically active non-toxic acid addition salt form by treatment with an acid; or conversely, converting the acid salt into the free base with alkali; and/or preparing stereochemically isomeric forms thereof.

## Patentansprüche

1. Eine Verbindung mit der Formel

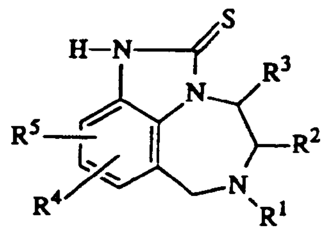

(I),

ein pharmazeutisch annehmbares Säureadditionssalz oder eine stereochemisch isomere Form hievon, worin

$R^1$ für $C_{1-6}$Alkyl, $C_{3-6}$Alkenyl, $C_{3-6}$Alkinyl, $C_{3-6}$Cycloalkyl oder durch Aryl oder $C_{3-6}$Cycloalkyl substituiertes $C_{1-6}$Alkyl steht;

$R^2$ Wasserstoff oder $C_{1-6}$Alkyl bedeutet;

$R^3$ Wasserstoff oder $C_{1-6}$Alkyl darstellt;

$R^4$ und $R^5$ jeweils unabhängig voneinander für Wasserstoff, $C_{1-6}$Alkyl, Halogen, Cyano, Nitro, Trifluormethyl, Hydroxy, $C_{1-6}$Alkyloxy, Amino oder Mono- oder Di ($C_{1-6}$alkylamino) stehen; und Aryl für Phenyl steht, das gegebenenfalls durch 1 bis 3 Substituenten, unabhängig voneinander ausgewählt unter $C_{1-6}$Alkyl, Halogen, Hydroxy, $C_{1-6}$Alkyloxy, Amino, Nitro und Trifluormethyl, substituiert ist.

2. Verbindung nach Anspruch 1, worin $R^1$ für $C_{1-6}$Alkyl, $C_{3-6}$Alkenyl, $C_{3-6}$Alkinyl oder durch Aryl oder $C_{3-6}$Cycloalkyl substituiertes $C_{1-6}$Alkyl steht; $R^4$ und $R^5$ jeweils unabhängig voneinander Wasserstoff, $C_{1-6}$Alkyl, Halogen, Cyano, Nitro, Trifluormethyl, Hydroxy oder $C_{1-6}$Alkyloxy bedeuten.

3. Verbindung nach Anspruch 2, worin $R^1$ für $C_{3-6}$Alkyl, $C_{3-6}$Alkenyl oder durch $C_{3-6}$Cycloalkyl substituiertes $C_{1-6}$Alkyl steht; $R^2$ $C_{1-6}$Alkyl bedeutet; $R^5$ Wasserstoff darstellt.

4. Verbindung nach Anspruch 3, worin $R^1$ für $C_{3-6}$Alkyl, $C_{3-6}$Alkenyl oder ($C_{3-6}$Cycloalkyl )methyl steht; $R^2$ Methyl bedeutet; $R^3$ Wasserstoff darstellt; $R^4$ Wasserstoff, Methyl, Halogen, Cyano, Nitro oder Trifluormethyl bedeutet.

5. Verbindug nach Anspruch 4, worin $R^1$ Propyl, 2-Propenyl, 2-Butenyl, 2-Methyl-2-butenyl, 3-Methyl-2-Butenyl, 2, 3-Dimethyl-2-butenyl oder Cyclopropylmethyl darstellt; $R^4$ für Wasserstoff, Methyl oder Chlor steht.

6. Verbindung nach Anspruch 1 , worin die Verbindung unter 4, 5, 6, 7-Tetrahydro-5-methyl-6-propylimidazo-[ 4.5.1-jk] [ 1,4] benzodiazepin-2(1H)thion; (+)-(S)-4,5,6,7-Tetrahydro-5-methyl-6-(3-methyl-2-butenyl-)imidazo[4,5,1-jk] [ 1,4] benzodiazepin-2(1H)-thion; (+)-(S)-9-Chlor-4,5,6,7-tetrahydro-5-methyl-6-(3-methyl-2-butenyl)imidazo[ 4,5,1-jk] [ 1,4] benzodiazepin-2(1H)-thion; den pharmazeutisch annehmbaren Säureadditionssalzen hievon und den stereochemisch isomeren Formen hievon ausgewählt ist.

7. Eine Verbindung mit der Formel

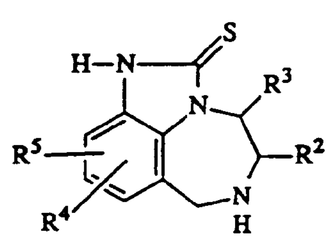

(V),

ein Säureadditionssalz oder eine stereochemish isomere Form hievon, worin $R^2$, $R^3$, $R^4$ und $R^5$ wie in Anspruch 1 definiert sind.

8. Eine Verbindung mit der Formel

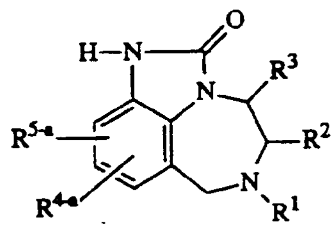

(IV-a),

ein pharmazeutisch annehmbares Säureadditionssalz oder eine stereochemisch isomere Form hievon, worin $R^1$, $R^2$ und $R^3$ wie in Anspruch 1 definiert sind; und

(a) $R^{4-a}$ und $R^{5-a}$ jeweils unabhängig voneinander $C_{1-6}$Alkyl, Halogen,Cyano, Nitro, Trifluormethyl, Hydroxy, $C_{1-6}$Alkyloxy, Amino oder Mono- oder Di ($C_{1-6}$alkyl)amino bedeutet, oder

(b) $R^{4-a}$ für Wasserstoff steht und $R^{5-a}$ Cyano, Nitro, Trifluormethyl, Hydroxy, $C_{1-6}$Alkyloxy, Amino oder Mono- oder Di($C_{1-6}$alkyl)amino darstellt.

**9.** Verbindung nach einem der Ansprüche 1 bis 6 und 8 zur Verwendung als ein Medikament.

**10.** Pharmazeutische Zusammensetzung , umfassend einen pharmazeutischen Träger und als wirksamen Bestandteil eine therapeutisch wirksame Menge einer Verbindung nach einem der Ansprüche 1 bis 6 und 8.

**11.** Verfahren zur Herstellung einer pharmazeutisch Zusammensetzung nach Anspruch 10, dadurch gekennzeichnet, daß eine therapeutisch wirksame Menge einer Verbindung nach einem der Ansprüche 1 bis 6 und 8 innig mit einem pharmazeutischen Träger vermischt wird.

**12.** Verfahren zur Herstellung einer Verbindung nach einem der Ansprüche 1 bis 6, gekennzeichnet durch

a) Kondensieren eines 9-Amino-2,3,4,5-tetrahydro-1H-1,4-benzodiazepins der Formel

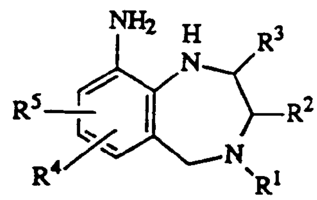

(II).

worin $R^1$, $R^2$, $R^3$, $R^4$ und $R^5$ wie in Formel (I) definiert sind, mit einem Reagens der Formel L-C(=S)-L (III), worin L eine Leaving-Gruppe bedeutet, in einem reaktionsinerten Lösungsmittel;

b) Thionieren eines 4,5,6,7-Tetrahydroimidazo[ 4,5,1-jk] [1,4] -benzodiazepin-2-ons der Formel

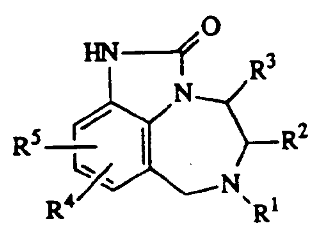

(IV).

worin $R^1$, $R^2$, $R^3$, $R^4$ und $R^5$ wie in Formel (I) definiert sind,

i) durch Umsetzung mit einem Halogenierungsmittel und Umwandlung des solcherart erhaltenen 2-Halogen-4, 5, 6, 7-tetrahydroimidazo[4,5,1-jk][1,4] benzodiazepins mit Thioharnstoff oder einem Alkalimetallthiosulfat in einem reaktionsinerten Lösungsmittel; oder

ii) durch Thionierung mit 2, 4-Bis(4-methoxyphenyl)-1,3-dithia-2,4-diphosphaten-2,4-disulfid oder

Phosphorpentasulfid in einem inerten Lösungsmittel;

c) N-Alkylieren eines Zwischenprodukts der Formel

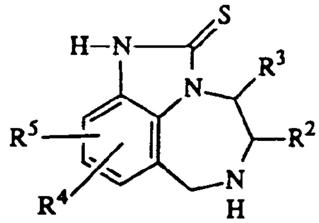

(V),

worin $R^2$, $R^3$, $R^4$ und $R^5$ wie in Formel (I) definiert sind mit einem Reagens der Formel $R^1$-W (VI-a), worin W eine reaktionsfähige Leaving-Gruppe bezeichnet und $R^1$ wie unter Formel (I) definiert ist, in einem reaktionsinerten Lösungsmittel;

d) N-Alkylieren eines Zwischenproduckts der Formel

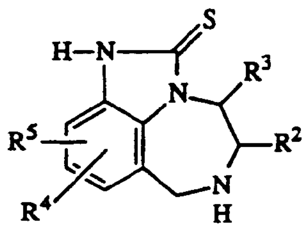

(V)

(V),

worin $R^2$, $R^3$, $R^4$ und $R^5$ wie in Formel (I) definiert sind, mit einem Keton oder Aldehyd der Formel $R^{1-b}$=O(VI-b), worin $R^{1-b}$ einen geminalen zweiwertigen Rest, abgeleitet von $R^{1-a}$-H, bedeutet, wobei $R^{1-a}$ für $C_{1-6}$Alkyl, $C_{3-6}$Cycloalkyl oder durch Aryl oder $C_{3-6}$Cycloalkyl substituiertes $C_{1-6}$Alkyl steht, worin zwei geminale Wasserstoffatome durch =O ersetzt sind, in einem reaktionsinerten Lösungsmittel unter Ausbildung einer Verbindung der Formel

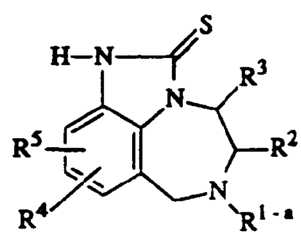

(I-a);

e) Thiatieren eines Tetrahydroimidazo[ 4,5,1-jk][ 1,4] benzodiazepins der Formel

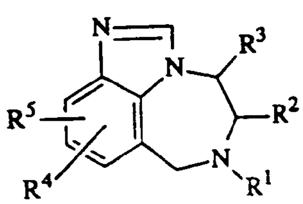

(VII),

worin $R^1$, $R^2$, $R^3$, $R^4$ und $R^5$ wie in Formel (I) definiert sind, mit elementarem Schwefel bei erhöhter Temperatur;

f) Reduzieren und Thiocarbonylieren eines 9-Nitrobenzodiazepins der Formel

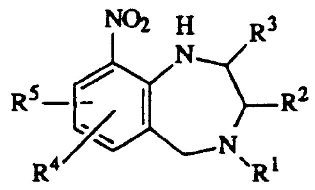

(VIII),

worin $R^1$, $R^2$, $R^3$, $R^4$ und $R^5$ wie in Formel (I) definiert sind, in Anwensenheit eines Alkalimetallsulfids oder -hydrogensulfids und von Schwefelkohlenstoff;

g) Cyclisieren eines Benzimidazol-2-thions der Formel

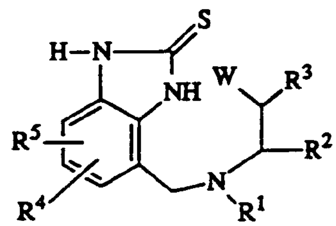

(IX),

worin $R^1$, $R^2$, $R^3$, $R^4$ und $R^5$ wie in Formel (I) definiert sind und W eine reaktionsfähige Leaving-Gruppe bezeichnet, in einem reaktionsinerten Lösungsmittel, und, falls erwünscht, Überführen der Verbindungen der Formel (I) in eine therapeutisch wirksame, nicht-toxische Säureadditionssalzform durch Behandlung mit einer Säure; oder umgekehrt Überführen des Säuresalzes in die freie Base mit Alkali; und-/oder Bereiten stereochemisch isomerer Formen hiervon.

**13.** Verfahren zur Herstellung einer Verbindung nach Anspruch 7, gekennzeichnet durch

a) Kondensieren eines 9-Amino-2,3,4,5-tetrahydro-1H-1,4-benzodiazepins der Formel

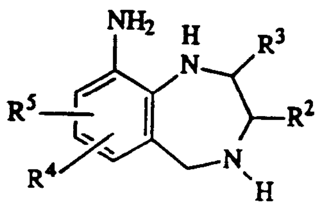

(II-a),

worin $R^2$, $R^3$, $R^4$ und $R^5$ wie in Formel (I) definiert sind, mit einem Reagens der Formel L-C(=S)-L (III), worin L eine Leavinggruppe bezeichnet, in einem reaktionsinerten Lösungsmittel;

b) Thionieren eines 4,5,6,7-Tetrahydroimidazo[4,5,1-jk][1,4]-benzodiazepin-2-ons der Formel

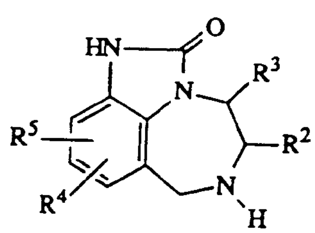

(IV-b),

worin $R^2$, $R^3$, $R^4$ und $R^5$ wie in Formel (I) definiert sind,

i) durch Umsetzung mit einem Halogenierungsmittel und Umwandlung des solcherart erhaltenen 2-Halogen-4,5,6,7-tetrahydroimidazo[4,5,1-jk][1,4]benzodiazepins mit Thioharnstoff oder einem Alkalimetallthiosulfat in einem reaktionsinerten Lösungsmittel; oder

ii) durch Thionierung mit 2,4-Bis(4-methoxyphenyl)-1,3-dithia-2,4-diphosphetan-2,4-disulfid oder Phosphorpentasulfid in einem inerten Lösungsmittel;

c) Thiatieren eines Tetrahydroimidazo[ 4,5,1-jk] [ 1,4] benzodiazepins der Formel

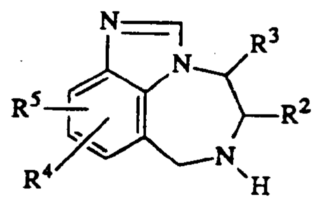

(VII-a),

worin $R^2$, $R^3$, $R^4$ und $R^5$ wie in Formel (I) definiert sind, mit elementarem Schwefel bei erhöhter Temperatur;

d) Reduzieren und Thiocarbonylieren eines 9-Nitrobenzodiazepins der Formel

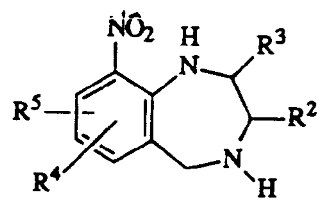

(VIII-a),

worin $R^2$, $R^3$, $R^4$ und $R^5$ wie in Formel (I) definiert sind, in Anwesenheit eines Alkalimetallsulfids oder -hydrogensulfids und von Schwefelkohlenstoff;

e) Cyclisieren eines Benzimidazol-2-thions der Formel

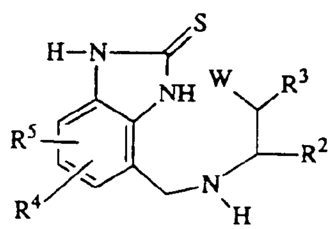

(IX-a),

worin $R^2$, $R^3$, $R^4$ und $R^5$ wie in Formel (I) definiert sind und W eine reaktionsfähige Leaving-Gruppe bezeichnet, in einem reaktionsinerten Lösungsmittel, und, falls erwünscht, Überführen der Verbindungen der Formel (I) in eine therapeutisch wirksame, nicht-toxische Säureadditionssalzform durch in Behandlung mit einer Säure; oder umgekehrt, Überführen des Säuresalzes in die freie Base mit Alkali; und/oder Bereiten stereochemisch isomerer Formen hievon.

**14.** Verfahren zur Herstellung einer Verbindung nach Anspruch 8, gekennzeichnet durch

a) Kondensieren eines 9-Amino-2,3,4,5-terahydro-1H-1,4-benzodiazepins der Formel

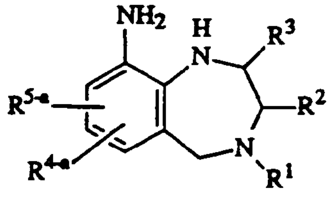

(II-b),

worin $R^1$, $R^2$, $R^3$, $R^{4-a}$ und $R^{5-a}$ wie in Anspruch 8 definiert sind, mit einem Reagens der Formel L-C(=O)-L (LVIII), worin L eine Leavinggruppe bezeichnet, in einem reaktionsinerten Lösungsmittel;
b) N-Alkylieren eines Zwischenprodukts der Formel

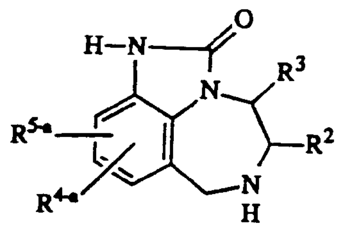

(IV-b-1),

worin $R^2$, $R^3$, $R^{4-a}$ und $R^{5-a}$ wie in Anspruch 8 definiert sind, mit einem Reagens der Formel $R^1$-W (VI-a), worin W eine reaktionsfähige Leaving-Gruppe bezeichnet und $R^1$ wie unter Formel (I) definiert ist, in einem reaktionsinerten Lösungsmittel;
c) N-Alkylieren eines Zwischenprodukts der Formel

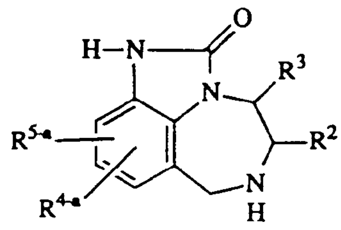

(IV-b-1),

worin $R^2$, $R^3$, $R^{4-a}$ und $R^{5-a}$ wie in Anpruch 8 definiert sind, mit einem Keton oder Aldehyd der Formel $R^{1-b}$=O (VI-b), worin $R^{1-b}$ einen geminalen zweiwertigen Rest, abgeleitet von $R^{1-a}$-H, bedeutet, worin $R^{1-a}$ für $C_{1-6}$Alkyl, $C_{3-6}$Cycloalkyl oder durch Aryl oder $C_{3-6}$Cycloalkyl substituiertes $C_{1-6}$Alkyl steht, worin zwei geminale Wasserstoffatome durch =O ersetzt sind, in einem reaktionsinerten Lösungsmittel unter Ausbildung einer Verbindung der Formel

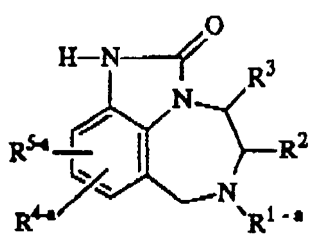

(IV-a-1);

und gewünschtenfalls Überführen der Verbindungen der Formel (IV-a) in eine therapeutisch wirksame nicht-toxische Säureadditionssalzform durch Behandlung mit einer Säure; oder umgekehrt, Überführen des Säuresalzes in die freie Base mit Alkali; und/oder Bereiten stereochemisch isomerer Formen hievon.

## Revendications

1. Composé de formule

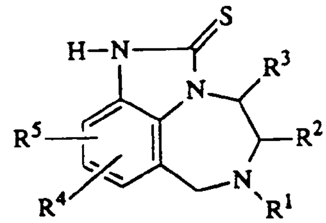

(I),

un sel d'addition d'acide pharmaceutiquement acceptable ou une forme isomère stéréochimique de celui-ci, dans laquelle formule

$R^1$ est un alkyle en $C_{1-6}$, un alcényle en $C_{3-6}$, un alcynyle en $C_{3-6}$, un cycloalkyle en $C_{3-6}$ ou un alkyle en $C_{1-6}$ substitué avec un aryle ou avec un cycloalkyle en $C_{3-6}$ ;

$R^2$ est un hydrogène ou un alkyle en $C_{1-6}$ ;

$R^3$ est un hydrogène ou un alkyle en $C_{1-6}$ ;

$R^4$ et $R^5$ sont chacun indépendamment un hydrogène, un alkyle en $C_{1-6}$, un halogéno, un cyano, un nitro, un trifluorométhyle, un hydroxy, un alkyloxy en $C_{1-6}$, un amino ou un mono- ou di(alkyl en $C_{1-6}$)amino ; et

un aryle est un phényle facultativement substitué avec 1 à 3 substituants choisis indépendamment parmi un alkyle en $C_{1-6}$, un halogéno, un hydroxy, un alkyloxy en $C_{1-6}$, un amino, un nitro et un trifluorométhyle.

2. Composé selon la revendication 1, dans lequel $R^1$ est un alkyle en $C_{1-6}$, un alcényle en $C_{3-6}$, un alcynyle en $C_{3-6}$ ou un alkyle en $C_{1-6}$ substitué avec un aryle ou avec un cycloalkyle en $C_{3-6}$ ; et $R^4$ et $R^5$ sont chacun indépendamment un hydrogène, un alkyle en $C_{1-6}$, un halogéno, un cyano, un nitro, un trifluorométhyle, un hydroxy ou un alkyloxy en $C_{1-6}$.

3. Composé selon la revendication 2, dans lequel $R^1$ est un alkyle en $C_{3-6}$, un alcényle en $C_{3-6}$ ou un alkyle en $C_{1-6}$ substitué avec un cycloalkyle en $C_{3-6}$ ; $R^2$ est un alkyle en $C_{1-6}$ ; et $R^5$ est un hydrogène.

4. Composé selon la revendication 3, dans lequel $R^1$ est un alkyle en $C_{3-6}$, un alcényle en $C_{3-6}$ ou un (cycloalkyl en $C_{3-6}$) méthyle ; $R^2$ est un méthyle ; $R^3$ est un hydrogène ; et $R^4$ est un hydrogène, un méthyle, un halogéno, un cyano, un nitro ou un trifluorométhyle.

5. Composé selon la revendication 4, dans lequel $R^1$ est un propyle, un 2-propényle, un 2-butényle, un 2-méthyl-2-butényle, un 3-méthyl-2-butényle, un 2,3-diméthyl-2-butényle ou un cyclopropylméthyle ; et $R^4$ est un hydrogène, un méthyle ou un chloro.

6. Composé selon la revendication 1 qui est choisi parmi la 4,5,6,7-tétrahydro-5-méthyl-6-propylimidazo[4,5,1-jk][1,4]benzodiazépine-2(1H)-thione ; la (+)-(S)-4,5,6,7-tétrahydro-5-méthyl-6-(3-méthyl-2-butényl)imidazo[4,5,1-jk][1,4]benzodiazépine-2(1H)thione ; la (+)-(S)-9-chloro-4,5,6,7-tétrahydro-5-méthyl-6-(3-méthyl-2-butényl)imidazo[4,5,1-jk][1,4]benzodiazépine-2(1H)-thione ; leurs sels d'addition d'acides pharmaceutiquement acceptables et leurs formes isomères stéréochimiques.

7. Composé de formule

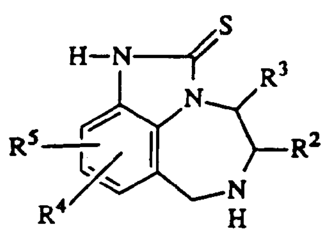

(V)

un sel d'addition d'acide ou une forme isomère stéréochimique de celui-ci, où

$R^2$, $R^3$, $R^4$ et $R^5$ sont comme définis dans la revendication 1.

8. Composé de formule

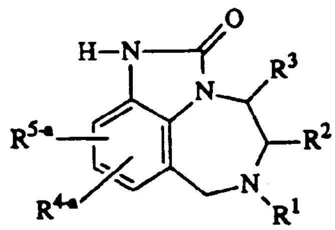

(IV-a)

un sel d'addition d'acide pharmaceutiquement acceptable ou une forme isomère stéréochimique de celui-ci, où $R^1$, $R^2$ et $R^3$ sont comme définis dans la revendication 1 ; et

(a) $R^{4-a}$ et $R^{5-a}$ représentent chacun indépendamment un alkyle en $C_{1-6}$, un halogéno, un cyano, un nitro, un trifluorométhyle, un hydroxy, un alkyloxy en $C_{1-6}$, un amino ou un mono- ou di(alkyl en $C_{1-6}$)amino, ou

(b) $R^{4-a}$ est un hydrogène et $R^{5-a}$ est un cyano, un nitro, un trifluorométhyle, un hydroxy, un alkyloxy en $C_{1-6}$, un amino ou un mono- ou di(alkyl en $C_{1-6}$)amino.

**9.** Composé selon l'une quelconque des revendications 1 à 6 et 8 pour l'emploi comme médicament.

**10.** Composition pharmaceutique comprenant un véhicule pharmaceutique et, comme ingrédient actif, une quantité thérapeutique efficace d'un composé selon l'une quelconque des revendications 1 à 6 et 8.

**11.** Procédé de préparation d'une composition pharmaceutique selon la revendication 10, caractérisé en ce que l'on mélange intimement une quantité thérapeutique efficace d'un composé selon l'une quelconque des revendications 1 à 6 et 8 avec un véhicule pharmaceutique.

**12.** Procédé pour préparer un composé selon l'une quelconque des revendications 1 à 6, caractérisé par

a) la condensation d'une 9-amino-2,3,4,5-tétrahydro-1H-1,4-benzodiazépine de formule

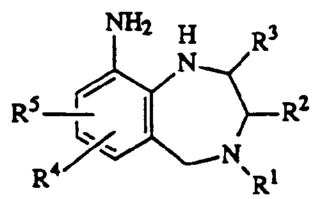

(II)

dans laquelle $R^1$, $R^2$, $R^3$, $R^4$ et $R^5$ sont comme définis pour la formule (I) avec un composé réagissant de formule L-C(=S)-L (III), dans laquelle L est un groupe labile, dans un solvant inerte vis-à-vis de la réaction,

b) la thionation d'une 4,5,6,7-tétrahydro-imidazo[4,5,1-jk] [1,4]benzodiazépine-2-one de formule

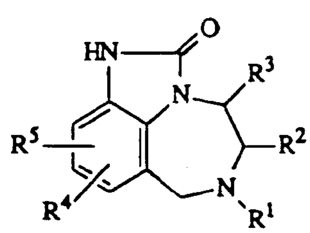

(IV)

dans laquelle $R^1$, $R^2$, $R^3$, $R^4$ et $R^5$ sont comme définis pour la formule (I)

i) par réaction avec un agent d'halogénation et conversion de la 2-halogéno-4,5,6,7-tétrahydro-imidazo[4,5,1-jk][1,4] benzodiazéplne ainsi obtenue avec la thio-urée ou un thiosulfate de métal alcalin dans un solvant inerte dans la réaction ; ou

ii) par thionation avec le 2,4-bis(4-méthoxyphényl)-1,3-dithia-2,4-diphosphétanne-2,4-disulfure ou le pentasulfure de phosphore dans un solvant inerte ;

c) la N-alkylation d'un intermédiaire de formule

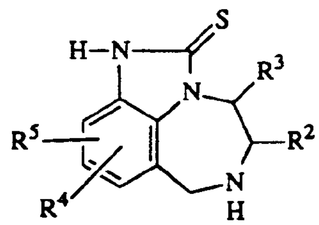

(V)

dans laquelle $R^2$, $R^3$, $R^4$ et $R^5$ sont comme définis pour la formule (I), avec un composé réagissant de formule $R^1$-W (VI-a) dans laquelle W représente un groupe labile réactif et $R^1$ est comme défini pour la formule (I), dans un solvant inerte dans la réaction ;

d) la N-alkylation d'un Intermédiaire de formule

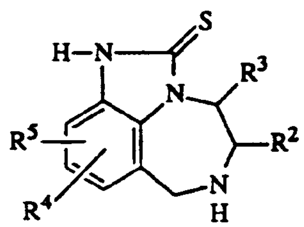

(V)

dans laquelle $R^2$, $R^3$, $R^4$ et $R^5$ sont comme définis pour la formule (I), avec une cétone ou un aldéhyde de formule $R^{1-b}$=O (VI-b) dans laquelle $R^{1-b}$ représente un radical bivalent géminal dérivé de $R^{1-a}$-H, $R^{1-a}$ étant un alkyle en $C_{1-6}$, un cycloalkyle en $C_{3-6}$ ou un alkyle en $C_{1-6}$ substitué avec un aryle ou avec un cycloalkyle en $C_{3-6}$, où deux atomes d'hydrogène géminaux sont remplacés par =O, dans un solvant inerte vis-à-vis de la réaction, pour obtenir un composé de formule

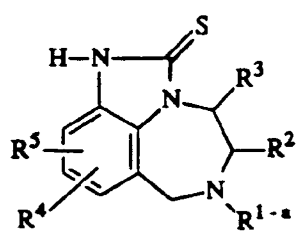

(I-a);

e) la thiation d'une tétrahydro-imidazo[4,5,1-jk][1,4] benzodiazépine de formule

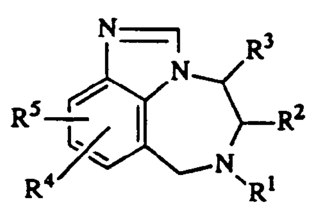

(VII)

dans laquelle $R^1$, $R^2$, $R^3$, $R^4$ et $R^5$ sont comme définis pour la formule (I), avec du soufre élémentaire à température élevée ;

f) la réduction et la thiocarbonylation d'une 9-nitrobenzodiazépine de formule

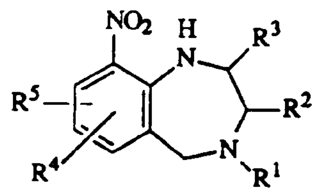

(VIII)

dans laquelle $R^1$, $R^2$, $R^3$, $R^4$ et $R^5$ sont comme définis pour la formule (I), en présence d'un sulfure ou hydrogénosulfure de métal alcalin et de disulfure de carbone ;

g) la cyclisation d'une benzimidazole-2-thione de formule

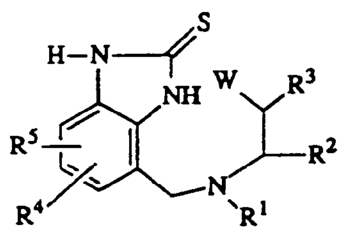

(IX)

dans laquelle $R^1$, $R^2$, $R^3$, $R^4$ et $R^5$ sont comme définis pour la formule (I) et W représente un groupe labile réactif, dans un solvant Inerte dans la réaction et, si on le désire, la conversion des composés de formule (I) en un sel d'addition d'acide non toxique par traitement avec un acide ; ou, inversement, la conversion du sel d'acide en la base libre avec un alcali ; et/ou la préparation des formes isomères stéréochimiques correspondantes.

**13.** Procédé pour préparer un composé selon la revendication 7, caractérisé par

a) la condensation d'une 9-amino-2,3,4,5-tétrahydro-1H-1,4-benzodiazépine de formule

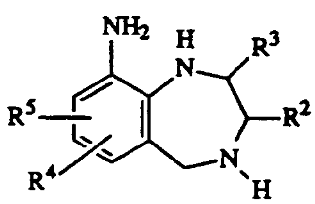

(II-a)

dans laquelle $R^2$, $R^3$, $R^4$ et $R^5$ sont comme définis pour la formule (I), avec un composé réagissant de formule L-C(=S)-L (III), dans laquelle L est un groupe labile, dans un solvant inerte dans la réaction ;

b) la thionation d'une 4,5,6,7-tétrahydro-imidazo[4,5,1-jk] [1,4]benzodiazépine-2-one de formule

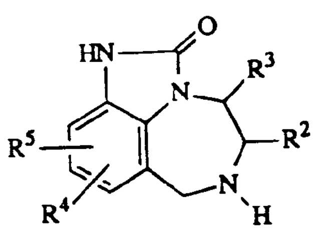

(IV-b)

dans laquelle $R^2$, $R^3$, $R^4$ et $R^5$ sont comme définis pour la formule (I)

i) par réaction avec un agent d'halogénation et conversion de la 2-halogéno-4,5,6,7-tétrahydro-imidazo[4,5,1-jk][1,4] benzodiazépine ainsi obtenue avec la thio-urée ou un thiosulfate de métal alcalin dans un solvant inerte dans la réaction, ou

ii) par thionation avec le 2,4-bis(4-méthoxyphényl)-1,3-dithia-2,4-diphosphétanne-2,4-disulfure ou le pentasulfure de phosphore dans un solvant inerte ;

c) la thiation d'une tétrahydro-imidazo[4,5,1-jk][1,4] benzodiazépine de formule

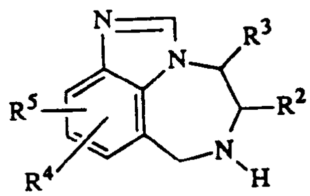

(VII-a)

dans laquelle $R^2$, $R^3$, $R^4$ et $R^5$ sont comme définis pour la formule (I), avec du soufre élémentaire à température élevée ;

d) la réduction et la thiocarbonylation d'une 9-nitrobenzodiazépine de formule

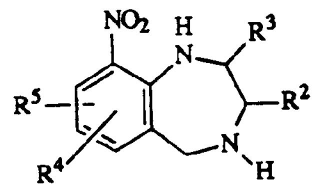

(VIII-a)

dans laquelle $R^2$, $R^3$, $R^4$ et $R^5$ sont comme définis pour la formule (I), en présence d'un sulfure ou d'un hydrogénosulfure de métal alcalin et de disulfure de carbone ;

e) la cyclisation d'une benzimidazole-2-thione de formule

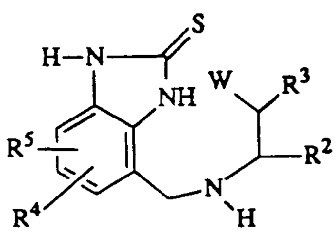

(IX-a)

dans laquelle $R^2$, $R^3$, $R^4$ et $R^5$ sont comme définis pour la formule (I) et W représente un groupe labile réactif, dans un solvant inerte dans la réaction et, si on le désire, la conversion des composés de formule (I) en un sel d'addition non toxique à activité thérapeutique par traitement avec un acide ; ou, inversement, la conversion du sel d'acide en la base libre avec un alcali ; et/ou la préparation des formes isomères stéréochimiques correspondantes.

**14.** Procédé pour préparer un composé selon la revendication 8, caractérisé par

a) la condensation d'une 9-amino-2,3,4,5-tétrahydro-1H-1,4-benzodiazépine de formule

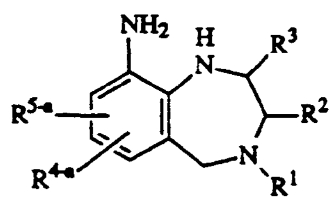

(II-b)

dans laquelle $R^1$, $R^2$, $R^3$, $R^4$ et $R^5$ sont comme définis dans la revendication 8, avec un composé réagissant de formule L-C(=O)-L (LVIII), dans laquelle L est un groupe labile, dans un solvant inerte dans la réaction ;

b) la N-alkylation d'un intermédiaire de formule

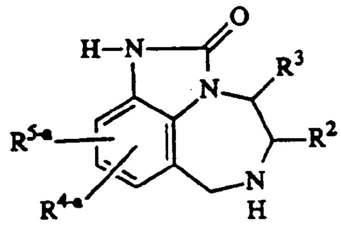

(IV-b-1)

dans laquelle $R^2$, $R^3$, $R^{4-a}$ et $R^{5-a}$ sont comme définis dans la revendication 8, avec un composé réagissant de formule $R^1$-W (VI-a), dans laquelle W représente un groupe labile réactif et $R^1$ est comme défini pour la formule (I), dans un solvant inerte dans la réaction ;

c) la N-alkylation d'un intermédiaire de formule

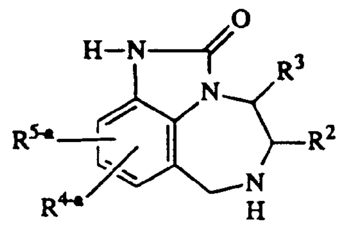

(IV-b-1)

dans laquelle $R^2$, $R^3$, $R^{4-a}$ et $R^{5-a}$ sont comme définis dans la renvendication 8, avec une cétone ou un aldéhyde de formule $R^{1-b}$=O (VI-b), dans laquelle $R^{1-b}$ représente un radical bivalent géminal dérivé de $R^{1-a}$-H, $R^{1-a}$ étant un alkyle en $C_{1-6}$, un cycloalkyle en $C_{3-6}$ ou un alkyle en $C_{1-6}$ substitué avec un aryle ou avec un cycloalkyle en $C_{3-6}$, où les deux atomes d'hydrogène géminaux sont remplacés par =O, dans un solvant inerte dans la réaction, pour former un composé de formule

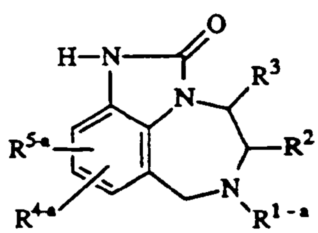

(IV-a-1);

et, si on le désire, la conversion des composés de formule (IV-a) en un sel d'addition d'acide non toxique à activité thérapeutique par traitement avec un acide ; ou, inversement, la conversion du sel d'acide en la base libre avec un alcali ; et/ou la préparation des formes isoméres stéréochimiques correspondantes.